(19) Europäisches Patentamt
European Patent Office
Office européen des brevets

(11) EP 2 118 306 B1

(12) **EUROPEAN PATENT SPECIFICATION**

(45) Date of publication and mention of the grant of the patent:
**13.02.2013 Bulletin 2013/07**

(21) Application number: **07857150.2**

(22) Date of filing: **28.12.2007**

(51) Int Cl.:
***C12Q 1/68*** *(2006.01)*

(86) International application number:
**PCT/EP2007/011458**

(87) International publication number:
**WO 2008/080620 (10.07.2008 Gazette 2008/28)**

(54) **METHODS FOR DETECTING METHICILLIN-RESISTANT S. AUREUS AS WELL AS PRIMERS, PROBES AND KITS FOR THE SAME**

VERFAHREN ZUM NACHWEIS METHICILLINRESISTENTER S. AUREUS SOWIE PRIMER, SONDEN UND KITS DAFÜR

PROCÉDÉS DE DÉTECTION DE S. AUREUS RÉSISTANTS À LA MÉTHICILLINE, AMORCES, SONDES ET TROUSSES À CET EFFET

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.12.2006 US 882799 P**
**23.04.2007 EP 07008206**

(43) Date of publication of application:
**18.11.2009 Bulletin 2009/47**

(73) Proprietors:
- **Roche Diagnostics GmbH**
**68305 Mannheim (DE)**
Designated Contracting States:
**DE**
- **Mayo Foundation for Medical Education and Research**
**Rochester, MN 55905 (US)**
- **F. Hoffmann-La Roche AG**
**4070 Basel (CH)**
Designated Contracting States:
**AT BE BG CH CY CZ DK EE ES FI FR GB GR HU IE IS IT LI LT LU LV MC MT NL PL PT RO SE SI SK TR**

(72) Inventors:
- **AICHINGER, Christian**
**81379 München (DE)**
- **REISER, Astrid**
**82377 Penzberg (DE)**
- **UHL, James, R.**
**Rochester, MN 55902 (US)**
- **COCKERILL, Franklin, R.**
**Rochester, MN 55905 (US)**

(74) Representative: **Bösl, Raphael Konrad**
**Isenbruck Bösl Hörschler LLP**
**Patentanwälte**
**Prinzregentenstrasse 68**
**81675 München (DE)**

(56) References cited:
**EP-A1- 1 529 847**
**WO-A-02/099034**
**WO-A-2006/111028**
**US-A1- 2007 054 296**

- **HOUGARDY ET AL: "Detection directe et rapide du portage de Staphylococcus aureus resistant a la methicilline par extraction automatisee de l'acide nucleique et PCR en temps reel" PATHOLOGIE ET BIOLOGIE, L'EXPANSION SCIENTIFIQUE FRANCAISE, PARIS, FR, vol. 54, no. 8-9, 14 November 2006 (2006-11-14), pages 477-481, XP005763192 ISSN: 0369-8114**
- **CUNY C ET AL: "PCR for the identification of methicillin-resistant Staphylococcus aureus (MRSA) strains using a single primer pair specific for SCCmec elements and the neighbouring chromosome-borne orfX." CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES OCT 2005, vol. 11, no. 10, October 2005 (2005-10), pages 834-837, XP002451789 ISSN: 1198-743X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 118 306 B1

- **ITO TERUYO ET AL: "Novel type V staphylococcal cassette chromosome mec driven by a novel cassette chromosome recombinase, ccrC." ANTIMICROBIAL AGENTS AND CHEMOTHERAPY JUL 2004, vol. 48, no. 7, July 2004 (2004-07), pages 2637-2651, XP002451790 ISSN: 0066-4804 cited in the application & ITO T ET AL: "Novel type V staphylococcal cassette chromosome mec driven by a novel cassette chromosome recombinase, ccrC" GENBANK, July 2004 (2004-07), XP003003510**
- **HULETSKY ANN ET AL: "Identification of methicillin-resistant Staphylococcus aureus carriage in less than 1 hour during a hospital surveillance program." CLINICAL INFECTIOUS DISEASES : AN OFFICIAL PUBLICATION OF THE INFECTIOUS DISEASES SOCIETY OF AMERICA 1 APR 2005, vol. 40, no. 7, 1 April 2005 (2005-04-01), pages 976-981, XP002451791 ISSN: 1537-6591**

SEQUENCE LISTING

<110> Roche Diagnostics GmbH, Mayo Foundation for Medical Education and Research, F. Hoffmann-La Roche AG, Roche Molecular system Inc.

<120> Methods for detecting methicillin-resistant S. aureus as well as primers, probes and kits for the same

<130> R64410PC

<160> 98

<170> PatentIn version 3.4

<210> 1
<211> 26
<212> DNA
<213> Artificial

<220>
<223> AR mec 1 fwd

<400> 1
gcaattcaca taaacctcat atgttc 26

<210> 2
<211> 24
<212> DNA

<213> Artificial

<220>
<223> AR mec 2 fwd

<400> 2
acctcatatg ttctgataca ttca 24

<210> 3
<211> 22
<212> DNA
<213> Artificial

<220>
<223> AR mec 3 fwd

<400> 3
gcaattcaca taaacctcat at 22

<210> 4
<211> 25
<212> DNA
<213> Artificial

<220>
<223> AR mec 4 fwd

<400> 4
cataacagca attcacataa acctc 25

<210> 5
<211> 22
<212> DNA
<213> Artificial

<220>
<223> AR mec 5 fwd

<400> 5
taacagcaat tcacataaac ct 22

<210> 6
<211> 27
<212> DNA
<213> Artificial

<220>
<223> AR mec 6 fwd

<400> 6
cgctattatt tacttgaaat gaaagac 27

<210> 7
<211> 21
<212> DNA
<213> Artificial

<220>

<223> AR mec 7 fwd

<400> 7
cttgaaatga aagactgcgg a 21

<210> 8
<211> 30
<212> DNA
<213> Artificial

<220>
<223> AR mec 8 fwd

<400> 8
ttgcttcact ataagtattc agtataaaga 30

<210> 9
<211> 24
<212> DNA
<213> Artificial

<220>
<223> AR mec 9 fwd

<400> 9
atttacttga aatgaaagac tgcg 24

<210> 10
<211> 29
<212> DNA
<213> Artificial

<220>
<223> AR mec 10 fwd

<400> 10
aaagaatatt tcgctattat ttacttgaa 29

<210> 11
<211> 29
<212> DNA
<213> Artificial

<220>
<223> AR mec 11 fwd

<400> 11
tcagtataaa gaatatttcg ctattattt 29

<210> 12
<211> 20
<212> DNA
<213> Artificial

<220>
<223> AR mec 12 fwd

<400> 12

tgaaatgaaa gactgcggag 20

<210> 13
<211> 27
<212> DNA
<213> Artificial

<220>
<223> JU1 fwd

<400> 13
aacctcatat gttctgatac attcaaa 27

<210> 14
<211> 28
<212> DNA
<213> Artificial

<220>
<223> JU2 fwd

<400> 14
tatgtcaaaa atcatgaacc tcattact 28

<210> 15
<211> 25
<212> DNA
<213> Artificial

<220>
<223> JU3 fwd

<400> 15
cataacagca attcacataa acctc 25

<210> 16
<211> 17
<212> DNA
<213> Artificial

<220>
<223> JU4 fwd

<400> 16
gactgcggag gctaact 17

<210> 17
<211> 18
<212> DNA
<213> Artificial

<220>
<223> JU5 fwd

<400> 17
atccctttat gaagcggc 18

<210> 18

<211> 30
<212> DNA
<213> Artificial

<220>
<223> AR mecA 3/1

<400> 18
gcaaggtata atccaatatt tcatatatgt 30

<210> 19
<211> 29
<212> DNA
<213> Artificial

<220>
<223> AR mecA 3/2

<400> 19
agttccataa tcaatataat ttgtacagt 29

<210> 20
<211> 23
<212> DNA
<213> Artificial

<220>
<223> AR mecA 3/3

<400> 20
acatcgtatg atattgcaag gta 23

<210> 21
<211> 26
<212> DNA
<213> Artificial

<220>
<223> AR mecA 3/4

<400> 21
ctttcattct ttcttgattc cattag 26

<210> 22
<211> 27
<212> DNA
<213> Artificial

<220>
<223> AR mecA 3/5

<400> 22
cactctataa acatcgtatg atattgc 27

<210> 23
<211> 30
<212> DNA
<213> Artificial

<220>
<223> AR mecA 3/6

<400> 23
ttcttaattt aattgtagtt ccataatcaa 30

<210> 24
<211> 30
<212> DNA
<213> Artificial

<220>
<223> AR mecA 3/7

<400> 24
aattatacac aacctaattt ttagttttat 30

<210> 25
<211> 29
<212> DNA
<213> Artificial

<220>
<223> AR mecA 3/8

<400> 25
aatttttagt tttatttatg atacgcttc 29

<210> 26
<211> 30
<212> DNA
<213> Artificial

<220>
<223> AR mecA 3/9

<400> 26
acacaaccta atttttagtt ttatttatga 30

<210> 27
<211> 30
<212> DNA
<213> Artificial

<220>
<223> AR mecA 3/10

<400> 27
tttattaaac actctataaa catcgtatga 30

<210> 28
<211> 31
<212> DNA
<213> Artificial

<220>
<223> AR mecA 3/13

<400> 28
ccacatctca ttaaattttt aaattataca c 31

<210> 29
<211> 30
<212> DNA
<213> Artificial

<220>
<223> AR mec 5/1

<400> 29
atattataca caatccgttt tttagtttta 30

<210> 30
<211> 29
<212> DNA
<213> Artificial

<220>
<223> AR mec 5/2

<400> 30
acacaatccg ttttttagtt ttatttatg 29

<210> 31
<211> 30
<212> DNA
<213> Artificial

<220>
<223> AR mec 5/3

<400> 31
ttctaattta tttaacataa aatcaatcct 30

<210> 32
<211> 33
<212> DNA
<213> Artificial

<220>
<223> AR mec 5/16

<400> 32
caatcctttt tatatttaaa atatattata cac 33

<210> 33
<211> 21
<212> DNA
<213> Artificial

<220>
<223> AR mec 1 rev

<400> 33
aggaaagatg ctatcttccg a 21

<210> 34
<211> 21
<212> DNA
<213> Artificial

<220>
<223> AR mec 2 rev

<400> 34
gaaagatgct atcttccgaa g 21

<210> 35
<211> 18
<212> DNA
<213> Artificial

<220>
<223> AR mec 3 rev

<400> 35
gatgctatct tccgaagg 18

<210> 36
<211> 29
<212> DNA
<213> Artificial

<220>
<223> AR mec 4 rev

<400> 36
gtcattacat tagaaataca aggaaagat 29

<210> 37
<211> 17
<212> DNA
<213> Artificial

<220>
<223> AR mec 5 rev

<400> 37 17
gccaacgaat actagcc 17

<210> 38
<211> 24
<212> DNA
<213> Artificial

<220>
<223> AR mec 6-2 rev

<400> 38
acgaatacta gccaaaatta aacc 24

<210> 39
<211> 25
<212> DNA

<213> Artificial

<220>
<223> AR mec 7 rev

<400> 39
cacaatccac agtcattaca ttaga 25

<210> 40
<211> 22
<212> DNA
<213> Artificial

<220>
<223> JU1 rev

<400> 40
caaggaaaga tgctatcttc cg 22

<210> 41
<211> 29
<212> DNA
<213> Artificial

<220>
<223> JU2 rev

<400> 41
agtcattaca ttagaaatac aaggaaaga 29

<210> 42
<211> 21
<212> DNA
<213> Artificial

<220>
<223> JU3 rev

<400> 42
aggaaagatg ctatcttccg a 21

<210> 43
<211> 21
<212> DNA
<213> Artificial

<220>
<223> JU4 rev

<400> 43
aggaaagatg ctatcttccg a 21

<210> 44
<211> 25
<212> DNA
<213> Artificial

<220>

<223> JU5 rev

<400> 44
acaatccaca gtcattacat tagaa 25

<210> 45
<211> 22
<212> DNA
<213> Artificial

<220>
<223> AR mec Fluo 1 w/o label

<400> 45
aagtcgcttt gcctttgggt ca 22

<210> 46
<211> 26
<212> DNA
<213> Artificial

<220>
<223> AR mec Fluo 2 w/o label

<400> 46
tacaaagtcg ctttgccttt gggtca 26

<210> 47
<211> 19
<212> DNA
<213> Artificial

<220>
<223> AR mec Fluo 3 w/o label

<400> 47
ggccgtttga tccgccaat 19

<210> 48
<211> 21
<212> DNA
<213> Artificial

<220>
<223> AR mec Fluo 4 w/o label

<400> 48
aagtcgcttt gcccttgggt a 21

<210> 49
<211> 20
<212> DNA
<213> Artificial

<220>
<223> AR mec Fluo 4-2 w/o label

<400> 49

aagtcgcttt gcccttgggt 20

<210> 50
<211> 22
<212> DNA
<213> Artificial

<220>
<223> AR mec Fluo 4-3 w/o label

<400> 50
aagtcgcttt gcccttgggt ca 22

<210> 51
<211> 19
<212> DNA
<213> Artificial

<220>
<223> AR mec Fluo 4-GV w/o label

<400> 51
aagtcgcttt gcccttggg 19

<210> 52
<211> 20
<212> DNA
<213> Artificial

<220>
<223> AR mec Fluo 4k w/o label

<400> 52
caagaattga accaacgcat 20

<210> 53
<211> 30
<212> DNA
<213> Artificial

<220>
<223> AR mec Fluo 5 w/o label

<400> 53
caatgacgaa tacatagtcg ctttgccctt 30

<210> 54
<211> 21
<212> DNA
<213> Artificial

<220>
<223> AR mec Fluo 6 w/o label

<400> 54
cgtttgatcc gccaatgacg a 21

<210> 55

<211> 22
<212> DNA
<213> Artificial

<220>
<223> AR mec Fluo 7 w/o label

<400> 55
gccaatcctt cggaagatag ca 22

<210> 56
<211> 18
<212> DNA
<213> Artificial

<220>
<223> AR mec Fluo UR w/o label

<400> 56
attaacacaa cccgcatc 18

<210> 57
<211> 19
<212> DNA
<213> Artificial

<220>
<223> JU1 probe 1 w/o label

<400> 57
gtcgctttgc ccttgggtc 19

<210> 58
<211> 20
<212> DNA
<213> Artificial

<220>
<223> JU2 probe 1 w/o label

<400> 58
tcgctttgcc cttgggtcat 20

<210> 59
<211> 19
<212> DNA
<213> Artificial

<220>
<223> JU3 probe 1 w/o label

<400> 59
ggccgtttga tccgccaat 19

<210> 60
<211> 22
<212> DNA
<213> Artificial

<220>
<223> JU4 probe 1 w/o label

<400> 60
gtccttgtgc aggccgtttg at 22

<210> 61
<211> 24
<212> DNA
<213> Artificial

<220>
<223> JU5 probe 1 w/o label

<400> 61
cttgggtcat gcgttggttc aatt 24

<210> 62
<211> 27
<212> DNA
<213> Artificial

<220>
<223> AR mec 640 3 w/o label

<400> 62
cgaatacaaa gtcgctttgc ccttggg 27

<210> 63
<211> 20
<212> DNA
<213> Artificial

<220>
<223> AR mec 640 4 w/o label

<400> 63
atgcgttggt tcaattcttg 20

<210> 64
<211> 20
<212> DNA
<213> Artificial

<220>
<223> AR mec 610 4-3 w/o label

<400> 64
gcgttggttc aattcttggg 20

<210> 65
<211> 20
<212> DNA
<213> Artificial

<220>
<223> AR mec 640 4k w/o label

<400> 65
acccaagggc aaagcgactt 20

<210> 66
<211> 24
<212> DNA
<213> Artificial

<220>
<223> AR mec 640 5 w/o label

<400> 66
ggtaatgcgt tggttcaatt cttg 24

<210> 67
<211> 25
<212> DNA
<213> Artificial

<220>
<223> AR mec 640 6 w/o label

<400> 67
acaaagtcgc tatgcccttg ggtca 25

<210> 68
<211> 29
<212> DNA
<213> Artificial

<220>
<223> AR mec 640 7 w/o label

<400> 68
ctttccttgt atttctaatg taatgactg 29

<210> 69
<211> 27
<212> DNA
<213> Artificial

<220>
<223> AR mec 640 UR w/o label

<400> 69
ttgatgtggg aatgtcattt tgctgaa 27

<210> 70
<211> 25
<212> DNA
<213> Artificial

<220>
<223> JU1 probe 2 w/o label

<400> 70
gcgttggttc aattcttggg ccaat 25

<210> 71
<211> 29
<212> DNA
<213> Artificial

<220>
<223> JU2 probe 2 w/o label

<400> 71
gttggttcaa ttcttgggcc aatccttcg 29

<210> 72
<211> 26
<212> DNA
<213> Artificial

<220>
<223> JU3 probe 1 w/o label

<400> 72
cgaatacaaa gtcgctttgc ccttgg 26

<210> 73
<211> 29
<212> DNA
<213> Artificial

<220>
<223> JU4 probe 2 w/o label

<400> 73
gccaatgacg aatacaaagt cgctttgcc 29

<210> 74
<211> 25
<212> DNA
<213> Artificial

<220>
<223> JU5 probe 2 w/o label

<400> 74
tgggccaatc cttcggaaga tagca 25

<210> 75
<211> 20
<212> DNA
<213> Artificial

<220>
<223> AR mec 610 4-MM2 w/o label

<400> 75
atgcgttggt tcgattcttg 20

<210> 76
<211> 21
<212> DNA

<213> Artificial

<220>
<223> AR mec 610 4-MM2-GV w/o label

<400> 76
catgcgttgg ttcgattctt g 21

<210> 77
<211> 255
<212> DNA
<213> Artificial

<220>
<223> orfx

<400> 77

ccacgcataa tcttaaatgc tctgtacact tgttcaatta acacaacccg catcatttga 60
tgtgggaatg tcattttgct gaatgatagt gcgtagttac tgcgttgtaa gacgtccttg 120
tgcaggccgt ttgatccgcc aatgacgaat acaaagtcgc tttgcccttg ggtcatgcgt 180
tggttcaatt cttgggccaa tccttcggaa gatagcatct ttccttgtat ttctaatgta 240
atgactgttg attgt 255

<210> 78
<211> 643
<212> DNA
<213> staphylococcus aureus

<400> 78

tttgcttcac tataagtatt cagtataaag aatatttcgc tattatttac ttgaaatgaa 60
agactgcgga ggctaactat gtcaaaaatc atgaacctca ttacttatga taagcttctt 120
aaaaacataa cagcaattca cataaacctc atatgttctg atacattcaa aatcccttta 180

tgaagcggct gaaaaaaccg catcatttga tatgcttctt aaaaacataa cagcaattca 240
cataaacctc atatgttctg atacattcaa aatcccttta tgaagcggct gaaaaaaccg 300
catcatttat gatatgcttc tccacgcata atcttaaatg ctctatacac ttgctcaatt 360
aacacaaccc gcatcatttg atgtgggaat gtcattttgc tgaatgatag tgcgtagtta 420
ctgcgttgta agacgtcctt gtgcaggccg tttgatccgc caatgacgaa tacaaagtcg 480
ctttgccctt gggtcatgcg ttggttcaat tcttgggcca atccttcgga agatagcatc 540
tttccttgta tttctaatgt aatgactgtg gattgtggtt taattttggc tagtattcgt 600
tggccttctt tttcttttac ttgctcaatt tctttgtcgc tca 643

<210> 79
<211> 513
<212> DNA
<213> Staphylococcus aureus

<400> 79

```
tttgcttcac tataagtatt cagtataaag aatatttcgc tattatttac ttgaaatgaa     60
agactgcgga ggctaactat gtcaaaaatc atgaacctca ttacttatga taagcttctt    120
aaaaacataa cagcaattca cataaacctc atatgttctg atacattcaa aatcccttta    180
tgaagcggct gaaaaaaccg catcatttat gatatgcttc tccacgcata atcttaaatg    240
ctctatacac ttgctcaatt aacacaaccc gcatcatttg atgtgggaat gtcattttgc    300
tgaatgatag tgcgtagtta ctgcgttgta agacgtcctt gtgcaggccg tttgatccgc    360
caatgacgaa tacaaagtcg ctttgccctt gggtcatgcg ttggttcaat tcttgggcca    420
atccttcgga agatagcatc tttccttgta tttctaatgt aatgactgtg gattgtggtt    480
taattttggc tagtattcgt tggccttctt ttt                                 513
```

<210> 80
<211> 411
<212> DNA
<213> Staphylococcus aureus

<400> 80

```
tttgcttcac tataagtatt cagtataaag aatatttcgc tattatttac ttgaaatgaa     60
agactgcgga ggctaactat gtcaaaaatc atgaacctca ttacttatga taagcttctc    120
cacgcataat cttaaatgct ctatacactt gctcaattaa cacaacccgc atcatttgat    180
gtgggaatgt cattttgctg aatgatagtg cgtagttact gcgttgtaag acgtccttgt    240
gcaggccgtt tgatccgcca atgacgaata caaagtcgct ttgcccttgg gtcatgcgtt    300
ggttcaattc ttgggccaat ccttcggaag atagcatctt tccttgtatt tctaatgtaa    360
tgactgtgga ttgtggttta attttggcta gtattcgttg gccttctttt t             411
```

<210> 81
<211> 595
<212> DNA
<213> Staphylococcus aureus

<220>
<221> misc_feature
<222> (39)..(39)
<223> n is a, c, g, or t

<400> 81

```
cattctttct tgattccatt agtttaaatt taaaatttnt catacaattt cttaatttaa     60
ttgtagttcc ataatcaata taatttgtac agttattata tattctagat catcaatagt    120
tgaaaaatgg tttattaaac actctataaa catcgtatga tattgcaagg tataatccaa    180
tatttcatat atgtaattcc tccacatctc attaaatttt taaattatac acaacctaat    240
ttttagtttt atttatgata cgcttctcca cgcataatct taaatgctct gtacacttgt    300
tcaattaaca caacccgcat catttgatgt gggaatgtca ttttgctgaa tgatagtgcg    360

tagttactgc gttgtaagac gtccttgtgc aggccgtttg atccgccaat gacgaataca    420
aagtcgcttt gcccttgggt catgcgttgg ttcaattctt gggccaatcc ttcggaagat    480
agcatctttc cttgtatttc taatgtaatg actgttgatt gtggtttgat tttggctagt    540
attcgttggc cttctttttc ttttacttgc tcaatttgct ttgtcgtctc atatt         595
```

<210> 82
<211> 388
<212> DNA
<213> Staphylococcus aureus

<400> 82

```
tccatctcta ctttattgtt ttcttcaaat attatctcgt aatttacctt gttcattaaa     60
caaaaaactg gataaaaaac cgcatcattt gtggtacgct tctccacgca taatcttaaa    120
tgctctgtac acttgttcaa ttaacacaac ccgcatcatt tgatgtggga atgtcatttt    180
gctgaatgat agtgcgtagt tactgcgttg taagacgtcc ttgtgcaggc cgtttgatcc    240
gccaatgacg aatacaaagt cgctttgccc ttgggtcatg cgttggttca attcttgggc    300
caatccttcg gaagatagca tctttccttg tatttctaat gtaatgactg ttgattgtgg    360
tttgattttg gctagtattc gttggcct                                       388
```

<210> 83
<211> 479
<212> DNA
<213> Staphylococcus aureus

<400> 83

```
agatgcctat aaactaacaa ttacaaatta ttattttgtg tttcacatta taatatatca     60
actagaatta attcttaata aaaagtaatc attaaaattt aataaactct gctttatatt    120
ataaaattac ggctgaaata accgcatcat ttatgatatg cttctcctcg cataatctta    180
aatgctctat acacttgttc aattaacaca acccgcatca tttgatgtgg gaatgtcatt    240
ttgctgaatg atagtgcgta gttactgcgt tgtaagacgt ccttgtgcag gccgtttgat    300
ccgccaataa cgaatacaaa gtcgctttgc ccttgggtca tgcgttggtt caattcttgg    360
gccaatcctt cggaagatag catctttcct tgtatttcta atgtaatgac tgtggattgt    420
ggtttgattt tggctagtat tcgttggcct tctttttctt ttacttgctc gatttcttt     479
```

<210> 84
<211> 475
<212> DNA
<213> Staphylococcus aureus

<400> 84

```
aaaaagaagt cgatttacac accatgtatt aaataatgga aattcttaat ctttacttgt     60
acctaaatta tcaaacttaa tattcacttt ttattcttca aagatttgag ctaatttaat    120
aattttctca tattttttag ttttatttgt ggtacgcttc tcctcgcata atcttaaatg    180
ctctatacac ttgttcaatt aacacaaccc gcatcatttg atgtgggaat gtcattttgc    240
tgaatgatag tgcgtagtta ctgcgttgta agacgtcctt gtgcaggccg tttgatccgc    300
caataacgaa tacaaagtcg ctttgccctt gggtcatgcg ttggttcaat tcttgggcca    360
atccttcgga agatagcatc tttccttgta tttctaatgt aatgactgtg gattgtggtt    420
tgattttggc tagtattcgt tggccttctt tttcttttac ttgctcgatt tcttt         475
```

<210> 85
<211> 477
<212> DNA

<213> Staphylococcus aureus

<400> 85

```
caaaaaatat atttacttta gtcaaatcat cttcactagt gtaattatcg aatgatttat     60
aactaacatt ttctaattta tttaacataa aatcaatcct ttttatattt aaaatatatt    120

atacacaatc cgttttttag ttttatttat gatacgcctc tccacgcata atcttaaatg    180
ctctatacac ttgttcaatt aacacaaccc gcatcatttg atgtgggaat gtcattttgc    240
taaatgatag tgcatagtta ctgcgttgta agacgtcctt gtgcaggccg tttgatccgc    300
caatgacgaa tacaaagtcg ctttgccctt gggtcatgcg ttggttcaat tcttgggcca    360
atccttcgga agatagcatc tttccttgta tttctaatgt aatgactgtg gattgtggtt    420
tgattttggc tagtattcgt tggccttctt tttcttttac ttgctcaatt tctttgt       477
```

<210> 86
<211> 643
<212> DNA
<213> Staphylococcus aureus

<220>
<221> misc_feature
<222> (21)..(21)
<223> n = c or g

<220>
<221> misc_feature
<222> (322)..(322)
<223> n = c or t

<220>
<221> misc_feature
<222> (323)..(323)
<223> n = c or g

<220>
<221> misc_feature
<222> (339)..(339)
<223> n = c or t

<220>
<221> misc_feature
<222> (345)..(345)
<223> n = a or g

<220>
<221> misc_feature
<222> (354)..(354)
<223> n = c or t

<220>
<221> misc_feature
<222> (387)..(387)
<223> n = a or g

<220>

<221> misc_feature
<222> (402)..(402)
<223> n = a or g

<220>
<221> misc_feature
<222> (414)..(414)
<223> n = a or g

<220>
<221> misc_feature
<222> (423)..(423)
<223> n = a or g

<220>
<221> misc_feature
<222> (471)..(471)
<223> n = a or t

<220>
<221> misc_feature
<222> (516)..(516)
<223> n = g or t

<220>
<221> misc_feature
<222> (570)..(570)
<223> n = g or t

<220>
<221> misc_feature
<222> (582)..(582)
<223> n = a or g

<220>
<221> misc_feature
<222> (584)..(584)
<223> n = t or none

<220>
<221> misc_feature
<222> (627)..(627)
<223> n = a or g

<400> 86

```
tttgcttcac tataagtatt nagtataaag aatatttcgc tattatttac ttgaaatgaa    60
agactgcgga ggctaactat gtcaaaaatc atgaacctca ttacttatga taagcttctt   120
aaaaacataa cagcaattca cataaacctc atatgttctg atacattcaa aatcccttta   180
tgaagcggct gaaaaaaccg catcatttga tatgcttctt aaaaacataa cagcaattca   240
cataaacctc atatgttctg atacattcaa aatcccttta tgaagcggct gaaaaaaccg   300
catcatttat gatatgcttc tnnacgcata atcttaaang ctctntacac ttgntcaatt   360
aacacaaccc gcatcatttg atgtggnaat gtcattttgc tnaatgatag tgcntagtta   420
ctncgttgta agacgtcctt gtgcaggccg tttgatccgc caatgacgaa nacaaagtcg   480
ctttgccctt gggtcatgcg ttggttcaat tcttgngcca atccttcgga agatagcatc   540
tttccttgta tttctaatgt aatgactgtn gattgtggtt tnantttggc tagtattcgt   600
tggccttctt tttcttttac ttgctcnatt tctttgtcgc tca                     643
```

<210> 87
<211> 23
<212> DNA
<213> Artificial

<220>
<223> TP mec 1

<400> 87
aagtcgcttt gcccttgggt cat 23

<210> 88
<211> 25
<212> DNA
<213> Artificial

<220>
<223> TP mec 2

<400> 88
tgctcaatta acacaacccg catca 25

<210> 89
<211> 33
<212> DNA
<213> Artificial

<220>
<223> MB mec 1

<400> 89
gccgcgctgc tcaattaaca caacccgcgc ggc 33

<210> 90
<211> 32
<212> DNA
<213> Artificial

<220>
<223> MB mec 2

<400> 90

gccgcgcatg cgttggttca attctgcgcg gc 32

<210> 91
<211> 18
<212> DNA
<213> Artificial

<220>
<223> MRSA IC 4

<400> 91
ccagcagaat gccaacca 18

<210> 92
<211> 20
<212> DNA
<213> Artificial

<220>
<223> MRSA direct RE2 fwd

<400> 92
tgaaatgaaa gactgcggag 20

<210> 93
<211> 31
<212> DNA
<213> Artificial

<220>
<223> MRSA direct RE3 fwd

<400> 93
ccacatctca ttaaattttt aaattataca c 31

<210> 94
<211> 33
<212> DNA
<213> Artificial

<220>
<223> MRSA direct RE7 fwd

<400> 94
caatcctttt tatatttaaa atatattata cac 33

<210> 95
<211> 22
<212> DNA
<213> Artificial

<220>
<223> MRSA direct rev

<400> 95
caaggaaaga tgctatcttc cg 22

<210> 96

<211> 19
<212> DNA
<213> Artificial

<220>
<223> MRSA direct Fluos w/o label

<400> 96
aagtcgcttt gcccttggg 19

<210> 97
<211> 21
<212> DNA
<213> Artificial

<220>
<223> MRSA direct Red 610 w/o label

<400> 97
catgcgttgg ttcgattctt g 21

<210> 98
<211> 20
<212> DNA
<213> Artificial

<220>
<223> MRSA direct IC Red 670 w/o label

<400> 98
ccagcagaat gccagccaat 20

## Description

The present invention relates to methods of detecting the presence or absence of methicillin-resistant *S. aureus* (MRSA) in a sample, the method comprising performing an amplifying step, a hybridizing step and a detecting step. Furthermore, the present invention relates to primers, probes and kits for the detection of MRSA.

*Staphylococcus aureus* (*S. aureus;* SA) is a bacterium, whose natural reservoir is mucous or wet/salty skin areas like the groin, anus or nose. In addition it inhabits wounds. Overall, the nose is the predominant environment for *S. aureus.* However, *S. aureus* can cause illnesses ranging from minor skin infections (such as pimples, boils, and cellulites) and abscesses, to severe diseases such as pneumonia, meningitis, endocarditis, toxic shock syndrome (TSS), septicemia and Multi Organ Dysfunction Syndrome (MODS). Each year some 500,000 patients in American hospitals contract a staphylococcal infection.

Today, *S. aureus* has become resistant to many commonly used antibiotics. In the UK, only 2 % of all *S. aureus* isolates are sensitive to penicillin with a similar picture in the rest of the world. The β-lactamase resistant-penicillins (methicillin, oxacillin, cloxacillin and flucloxacillin) were developed to treat penicillin-resistant *S. aureus* and are still used as first-line treatment. Methicillin was the first antibiotic in this class to be used (it was introduced in 1959), but only two years later, the first case of methicillin-resistant *S. aureus* (MRSA) was reported in England. MRSA may also be known as oxacillin-resistant *Staphylococcus aureus* (ORSA) and multiple-resistant *Staphylococcus aureus,* while non-methicillin resistant strains of *S. aureus* are sometimes called methicillin-susceptible *Staphylococcus aureus* (MSSA) if an explicit distinction must be made.

Despite its resistance, MRSA generally remained an uncommon finding even in hospital settings until the 1990's when the MRSA prevalence dramatically increased in hospitals where it is now endemic. Moreover, in the US there are increasing reports of outbreaks of MRSA colonisation and infection through skin contact in locker rooms and gymnasiums, even among healthy populations. MRSA also is becoming a problem in paediatrics (Johnson AP et al., 2005, J Antimicrob Chemother 56 (3): 455-62). As of early 2005, the number of deaths in the United Kingdom attributed to MRSA has been estimated by various sources to lie in the area of 3000 per year (Johnson, supra).

Infections caused by MRSA show a higher lethality rate and more severe symptoms, since treatment is restricted because of the resistance to only few antibiotics. First line treatment for MRSA is currently glycopeptide antibiotics (vancomycin and teicoplanin). However, there are number of problems with these antibiotics, mainly centered around the need for intravenous administration (there is no oral preparation available), toxicity and the need to monitor drug levels regularly by means of blood tests. There are also concerns that glycopeptide antibiotics do not penetrate very well into infected tissues (this is a particular concern with infections of the brain and meninges and in endocarditis). However, glycopeptides must not be used to treat methicillin-sensitive *S. aureus* as outcomes are inferior. Taken together, patients infected with MRSA do stay longer in hospital than patients just infected with SA.

Furthermore, unrecognized colonization with MRSA may lead to the distribution of MRSA from one patient to the other by replacing the flora of MRSA-uncolonized patients with MRSA. Colonization is eased by certain risk factors including e.g. previous antibiotic treatment, polymorbidity, and diabetes as well as previous stays in hospital.

From this follows that it is highly important to have a safe and reliable tool for the diagnosis and/or detection of MRSA.

Presently, the diagnosis of most skin infections is made by the pattern of symptoms and physical exam findings but it is not usually possible to know whether the infection is caused by *Staphylococcus* bacteria of any type or another bacterium, like e.g. group A Beta-hemolytic *Streptococcus* (*Streptococcus pyogenes*). To make a definitive diagnosis and to confirm that MRSA is the bacteria causing the infection, a culture can be done. However, diagnosing MRSA using a conventional culture requires 16-72 hours, causing a delay in treatment, significantly impairs patient outcomes and may facilitate outbreaks as well as increase hospital costs.

Phenotypic analyses, such as coagulase type, enterotoxin (SE) type, production of toxic shock syndrome toxin-1 and *in vitro* antibiotic susceptibility, have been used routinely for MRSA strain typing.

Additionally, genotypic analysis for MRSA strains has been established, providing a more detailed classification than phenotypic analysis. In particular, pulse-field gel electrophoresis (PFGE) using restriction fragments of total genomic DNA of MRSA is an excellent method of characterization.

Alternatively, multilocus sequence typing (MLST) may be used in order to characterize isolates of bacteria by using the sequences of internal fragments of seven housekeeping genes. MLST has been developed and validated for *S. aureus* (Enright et al. 2002, Proc. Nat. Acad. Sci. U.S.A. 99: 7687-7692) and provides a discriminatory method that allows related strains recovered in different countries to be readily identified.

EP 1529847 discloses a method for detecting MRSA using primers specific for the night junction of SSC mec.

Test kits have been developed in order to detect MRSA (e. g. IDI-MRSA assay, Becton Dickinson, USA) which is an in vitro diagnostic test for the direct detection of nasal colonization by methicillin-resistant *Staphylococcus aureus* (MRSA) to help prevent and control MRSA infections in healthcare settings.

However, this kit has a series of disadvantages which limit its usefulness. First, it does not detect MRSA of Staphylococcal Chromosomal Cassette (SCC) type V, which is important as SCC type V is a previously identified type, especially present in Asia and Australia. Second, its applicability is restricted for a higher number of probes. Third, the preparation of the samples is quite complicated or difficult. Fourth, the concept used for the controls is not state of the art. Moreover, the inhibition rate is quite high and, finally, a numerousness of primers and probes used is required, resulting in a diagnostic tool, which is prone to errors. Additionally, melting curve analysis which may be used by the FRET probe assay allows a higher specificity within the output signal compared to amplification curves. Therefore, one object of the present invention was to provide an alternative method for the detecting of MRSA, preferably an alternative method avoiding the above disadvantages.

The object of the present invention has been solved by providing a method of detecting the presence or absence of methicillin-resistant *S. aureus* (MRSA) in a sample, the method comprising

(a) performing an amplifying step comprising contacting the sample with a set of MRSA primers to produce an amplification product if MRSA is present in the sample,

(b) performing a hybridizing step comprising contacting the amplification product of step (a) with a pair of MRSA probes, wherein a first MRSA probe of the pair of MRSA probes is labeled with a donor fluorescent moiety and wherein a second MRSA probe of the pair of MRSA probes is labeled with a corresponding acceptor fluorescent moiety; and

(c) detecting the presence or absence of fluorescence resonance energy transfer (FRET) between the donor fluorescent moiety of the first MRSA probe and the acceptor fluorescent moiety of the second MRSA probe, wherein the presence of FRET is indicative of the presence of MRSA in the sample and wherein the absence of FRET is indicative of the absence of MRSA in the sample,

wherein the method is capable of detecting each of Staphylococcal Chromosomal Cassettes (SCC*mec)* types I to V of MRSA, and wherein the set of primers is as defined in appended claim 1.

The method may be used for fast and reliable detection of MRSA in a sample. Particularly, the method may be used in order to detect MRSA in a sample or diagnose an MRSA infection of an individual, such as a patient.

Accordingly, fast detection of MRSA improves the situation of patients suffering from MRSA, e.g. by earlier treatment, prevents outbreaks of MRSA by e.g. fast and strict application of barrier precautions for patients colonized or infected with MRSA in hospitals, and, therefore, reduces health care costs. Additionally, in preferred embodiments of the invention a limited number of primers, e.g. 3 or 4, may be used in order to detect members of all presently known SCC*mec* types, i.e. types I, II, III, IV and V which provides to a highly reliable tool for the detection of MRSA.

As detailed above, the method of the present invention is suitable for detection MRSA of all known Staphylococcal Chromosomal Cassettes (SCC*mec*) types, i.e. type I, II, III, IV and V. This is particularly important in order to choose the appropriate treatment e.g. for a patient infected with MRSA.

The methicillin resistance gene (*mecA*). *MecA* encodes an altered methicillin-resistant penicillin-binding protein (PBP2a or PBP2'), a penicillin binding protein with reduced affinity for β-lactam rings (the primary active-site of the β-lactam antibiotics such as penicillins, cephalosporins and carbapenems) (Guignard B et al., 2005, Curr Opin Pharmacol 5 (5): 479-89), that is not present in susceptible strains and is believed to have been acquired from a distantly related species. *MecA* is carried on a mobile genetic element, the Staphylococcal Chromosomal Cassette *mec* (SCC*mec*) of MRSA strains, of which five forms have been described that differ in size and genetic composition. SCC elements also occur in sensitive *S. aureus* but do not carry the *mecA* gene or carry a non functional *mecA* gene. Such strains are a major source of false positive results, because they do have the same right extremity junction.

However, MRSA detection from nasal specimen by detecting the *mecA* gene and a *S. aureus* specific gene leads to low positive predictive values (PPV) due to the presence of varying amounts of both non resistant SA and methicillin-resistant coagulase-negative staphylococci (MRCoNS). A combination of those is undistinguishable from MRSA, because of the presence of both targets. Depending on the prevalence of MRSA this situation leads up to 30 % false positive results. For a better PPV the chosen target needs to be unique for MRSA. The only target currently known is Staphylococcal Chromosomal Cassette (SCC*mec*), which amplifies the transposon integration site for the genetic element carrying the *mecA* gene.

*SCCmec,* the SCC element of MRSA (with functional *mec* A gene), is a transposon of a length of 16 kb - 67 kb integrated into the 3' portion of the open reading frame X from *S. aureus* (orfX) containing the *mecA* gene. *MecA* encodes the PBP2a conferring resistance to methicillin and its derivatives as well as to other antibiotics. OrfX has no defined function in *S. aureus* and is unique to SA. The integration of SCC*mec* creates a signature unique to MRSA.

SCC*mec* elements have two essential components, the *ccr* gene complex (*ccr*) and the *mec* gene complex *(mec).* The *ccr* gene complex is composed of *ccr* genes and surrounding open reading frames (ORFs), and the *mec* gene complex is composed of the *mecA* gene, regulatory genes, and insertion sequences upstream or downstream of *mecA.* Several *mec* and *ccr* allotypes have been found among SCC*mec* elements, which has led to the following classification (Ito et al, 2004, Antimicrob Agents Chemother. 48: 2637-2651):

- *SCCmec* of type I, with class B *mec* and type 1 *ccr;*
- *SCCmec* of type II, with class A *mec* and type 2 *ccr;*
- *SCCmec* of type III, with class A *mec* and type 3 *ccr;*
- *SCCmec* of type IV, with class B *mec* and type 2 *ccr;* and
- *SCCmec* of type V, with class C2 *mec* and type 5 *ccr.*

However, classification of MRSA and assignment to *SCCmec* type I to V is a phenotypic approach. Alternatively, an approach using different genotypes of MRSA may be used. One target for MRSA detection and classification based on genotypes may be the right extremity junction (RE) of the *SCCmec.* This alternative method of MRSA typing relates is therefore called RE (right extremity of *SCCmec)* typing. This typing method takes advantage of the polymorphism at the right extremity of *SCCmec* DNAs adjacent to the integration site among the three types of SCC*mec.* On the basis of the nucleic acid sequences of SEQ ID NO: 78 to 85 MRSA may be classified as follows:

RE2 Type A (GenBank gi|73537130|gb|DQ106887.1|; SEQ ID NO: 78)

TTTGCTTCACTATAAGTATTCAGTATAAAGAATATTTCGCTATTATTTACTTGAAATGAAA
GACTGCGGAGGCTAACTATGTCAAAAATCATGAACCTCATTACTTATGATAAGCTTCTTAA
AAACATAACAGCAATTCACATAAACCTCATATGTTCTGATACATTCAAAATCCCTTTATGA
AGCGGCTGAAAAAACCGCATCATTTGATATGCTTCTTAAAAACATAACAGCAATTCACATA
AACCTCATATGTTCTGATACATTCAAAATCCCTTTATGAAGCGGCTGAAAAAACCGCATCA
TTTATGATATGCTTCTCCACGCATAATCTTAAATGCTCTATACACTTGCTCAATTAACACA
ACCCGCATCATTTGATGTGGGAATGTCATTTTGCTGAATGATAGTGCGTAGTTACTGCGTT
GTAAGACGTCCTTGTGCAGGCCGTTTGATCCGCCAATGACGAATACAAAGTCGCTTTGCCC
TTGGGTCATGCGTTGGTTCAATTCTTGGGCCAATCCTTCGGAAGATAGCATCTTTCCTTGT
ATTTCTAATGTAATGACTGTGGATTGTGGTTTAATTTTGGCTAGTATTCGTTGGCCTTCTT
TTTCTTTTACTTGCTCAATTTCTTTGTCGCTCA

RE2_Type_B (GenBank gi|73537130|gb|DQ106887.1|; SEQ ID NO: 79)

TTTGCTTCACTATAAGTATTCAGTATAAAGAATATTTCGCTATTATTTACTTGAAATGAAA
GACTGCGGAGGCTAACTATGTCAAAAATCATGAACCTCATTACTTATGATAAGCTTCTTAA
AAACATAACAGCAATTCACATAAACCTCATATGTTCTGATACATTCAAAATCCCTTTATGA
AGCGGCTGAAAAAACCGCATCATTTATGATATGCTTCTCCACGCATAATCTTAAATGCTCT
ATACACTTGCTCAATTAACACAACCCGCATCATTTGATGTGGGAATGTCATTTTGCTGAAT
GATAGTGCGTAGTTACTGCGTTGTAAGACGTCCTTGTGCAGGCCGTTTGATCCGCCAATGA
CGAATACAAAGTCGCTTTGCCCTTGGGTCATGCGTTGGTTCAATTCTTGGGCCAATCCTTC
GGAAGATAGCATCTTTCCTTGTATTTCTAATGTAATGACTGTGGATTGTGGTTTAATTTTG
GCTAGTATTCGTTGGCCTTCTTTTT

RE2_Type_C (GenBank gi|57284222|gb|CP000046.1|; SEQ ID NO: 80)

TTTGCTTCACTATAAGTATTCAGTATAAAGAATATTTCGCTATTATTTACTTGAAATGAAA
GACTGCGGAGGCTAACTATGTCAAAAATCATGAACCTCATTACTTATGATAAGCTTCTCCA
CGCATAATCTTAAATGCTCTATACACTTGCTCAATTAACACAACCCGCATCATTTGATGTG
GGAATGTCATTTTGCTGAATGATAGTGCGTAGTTACTGCGTTGTAAGACGTCCTTGTGCAG
GCCGTTTGATCCGCCAATGACGAATACAAAGTCGCTTTGCCCTTGGGTCATGCGTTGGTTC
AATTCTTGGGCCAATCCTTCGGAAGATAGCATCTTTCCTTGTATTTCTAATGTAATGACTG
TGGATTGTGGTTTAATTTTGGCTAGTATTCGTTGGCCTTCTTTTT

RE3 (GenBank gi|23451317|gb|AF422696.1|; SEQ ID NO: 81)

CATTCTTTCTTGATTCCATTAGTTTAAATTTAAAATTTNTCATACAATTTCTTAATTTAAT
TGTAGTTCCATAATCAATATAATTTGTACAGTTATTATATATTCTAGATCATCAATAGTTG
AAAAATGGTTTATTAAACACTCTATAAACATCGTATGATATTGCAAGGTATAATCCAATAT
TTCATATATGTAATTCCTCCACATCTCATTAAATTTTTAAATTATACACAACCTAATTTTT
AGTTTTATTTATGATACGCTTCTCCACGCATAATCTTAAATGCTCTGTACACTTGTTCAAT
TAACACAACCCGCATCATTTGATGTGGGAATGTCATTTTGCTGAATGATAGTGCGTAGTTA
CTGCGTTGTAAGACGTCCTTGTGCAGGCCGTTTGATCCGCCAATGACGAATACAAAGTCGC
TTTGCCCTTGGGTCATGCGTTGGTTCAATTCTTGGGCCAATCCTTCGGAAGATAGCATCTT
TCCTTGTATTTCTAATGTAATGACTGTTGATTGTGGTTTGATTTTGGCTAGTATTCGTTGG
CCTTCTTTTTCTTTTACTTGCTCAATTTGCTTTGTCGTCTCATATT

RE4 (GenBank AY267374_; SEQ ID NO: 82)

TCCATCTCTACTTTATTGTTTTCTTCAAATATTATCTCGTAATTTACCTTGTTCATTAAAC
AAAAAACTGGATAAAAAACCGCATCATTTGTGGTACGCTTCTCCACGCATAATCTTAAATG
CTCTGTACACTTGTTCAATTAACACAACCCGCATCATTTGATGTGGGAATGTCATTTTGCT
GAATGATAGTGCGTAGTTACTGCGTTGTAAGACGTCCTTGTGCAGGCCGTTTGATCCGCCA
ATGACGAATACAAAGTCGCTTTGCCCTTGGGTCATGCGTTGGTTCAATTCTTGGGCCAATC
CTTCGGAAGATAGCATCTTTCCTTGTATTTCTAATGTAATGACTGTTGATTGTGGTTTGAT
TTTGGCTAGTATTCGTTGGCCT

RE5 (GenBank AY267381; SEQ ID NO: 83)

AGATGCCTATAAACTAACAATTACAAATTATTATTTTGTGTTTCACATTATAATATATCAA
CTAGAATTAATTCTTAATAAAAAGTAATCATTAAAATTTAATAAACTCTGCTTTATATTAT
AAAATTACGGCTGAAATAACCGCATCATTTATGATATGCTTCTCCTCGCATAATCTTAAAT
GCTCTATACACTTGTTCAATTAACACAACCCGCATCATTTGATGTGGGAATGTCATTTTGC
TGAATGATAGTGCGTAGTTACTGCGTTGTAAGACGTCCTTGTGCAGGCCGTTTGATCCGCC
AATAACGAATACAAAGTCGCTTTGCCCTTGGGTCATGCGTTGGTTCAATTCTTGGGCCAAT
CCTTCGGAAGATAGCATCTTTCCTTGTATTTCTAATGTAATGACTGTGGATTGTGGTTTGA
TTTTGGCTAGTATTCGTTGGCCTTCTTTTTCTTTTACTTGCTCGATTTCTTT

RE6 (GenBank AY267375; SEQ ID NO: 84)

AAAAAGAAGTCGATTTACACACCATGTATTAAATAATGGAAATTCTTAATCTTTACTTGTA
CCTAAATTATCAAACTTAATATTCACTTTTTATTCTTCAAAGATTTGAGCTAATTTAATAA
TTTTCTCATATTTTTTAGTTTTATTTGTGGTACGCTTCTCCTCGCATAATCTTAAATGCTC
TATACACTTGTTCAATTAACACAACCCGCATCATTTGATGTGGGAATGTCATTTTGCTGAA
TGATAGTGCGTAGTTACTGCGTTGTAAGACGTCCTTGTGCAGGCCGTTTGATCCGCCAATA
ACGAATACAAAGTCGCTTTGCCCTTGGGTCATGCGTTGGTTCAATTCTTGGGCCAATCCTT
CGGAAGATAGCATCTTTCCTTGTATTTCTAATGTAATGACTGTGGATTGTGGTTTGATTTT
GGCTAGTATTCGTTGGCCTTCTTTTTCTTTTACTTGCTCGATTTCTTT

RE7 (GenBank gi|49257031|dbj|AB121219.1|, SEQ ID NO: 85)

CAAAAAATATATTTACTTTAGTCAAATCATCTTCACTAGTGTAATTATCGAATGATTTATA
ACTAACATTTTCTAATTTATTTAACATAAAATCAATCCTTTTTATATTTAAAATATATTAT
ACACAATCCGTTTTTTAGTTTTATTTATGATACGCCTCTCCACGCATAATCTTAAATGCTC
TATACACTTGTTCAATTAACACAACCCGCATCATTTGATGTGGGAATGTCATTTTGCTAAA
TGATAGTGCATAGTTACTGCGTTGTAAGACGTCCTTGTGCAGGCCGTTTGATCCGCCAATG
ACGAATACAAAGTCGCTTTGCCCTTGGGTCATGCGTTGGTTCAATTCTTGGGCCAATCCTT
CGGAAGATAGCATCTTTCCTTGTATTTCTAATGTAATGACTGTGGATTGTGGTTTGATTTT
GGCTAGTATTCGTTGGCCTTCTTTTTCTTTTACTTGCTCAATTTCTTTGT

As detailed above, RE types describe the sequence variations of the right extremity junction of the SCC *mec* transposon. RE2 constitutes the majority of all RE types (representing SCC*mec* types I, II, III (some) and some IV). RE3 and RE7 represent SCC*mec* types III and V and SCC*mec* type V, respectively.

Accordingly, disclosed herein are particular sets of primers used in order to provide for detection of all SCC*mec* types. The set of primers may comprise

(i) a primer specific for MRSA type RE2, particularly a primer comprising or consisting of a nucleic acid sequence selected from the group consisting of

- 5'- GCA ATT CAC ATA AAC CTC ATA TGT TC -3' (AR *mec*1 fwd; SEQ ID NO: 1),
- 5'- ACC TCA TAT GTT CTG ATA CAT TCA -3' (AR *mec2* fwd; SEQ ID NO: 2),
- 5'- GCA ATT CAC ATA AAC CTC ATA T -3' (AR *mec3* fwd; SEQ ID NO: 3),
- 5'- CAT AAC AGC AAT TCA CAT AAA CCT C -3' (AR *mec4* fwd; SEQ ID NO: 4),
- 5'- TAA CAG CAA TTC ACA TAA ACC T -3' (AR *mec5* fwd; SEQ ID NO: 5),
- 5'- CGC TAT TAT TTA CTT GAA ATG AAA GAC -3' (AR *mec* 6 fwd; SEQ ID NO: 6),
- 5'- CTT GAA ATG AAA GAC TGC GGA -3' (AR *mec* 7 fwd; SEQ ID NO: 7),
- 5'- TTG CTT CAC TAT AAG TAT TCA GTA TAA AGA -3' (AR *mec* 8 fwd; SEQ ID NO: 8),
- 5'- ATT TAC TTG AAA TGA AAG ACT GCG -3' (AR *mec* 9 fwd; SEQ ID NO: 9),
- 5'- AAA GAA TAT TTC GCT ATT ATT TAC TTG AA -3' (AR *mec* 10 fwd; SEQ ID NO: 10),
- 5'- TCA GTA TAA AGA ATA TTT CGC TAT TAT TT -3' (AR *mec* 11 fwd; SEQ ID NO: 11),
- 5'- TGA AAT GAA AGA CTG CGG AG -3' (AR *mec* 12 fwd; SEQ ID NO: 12),
- 5'- AAC CTC ATA TGT TCT GAT ACA TTC AAA -3' (JU1 fwd; SEQ ID NO: 13),
- 5'- TAT GTC AAA AAT CAT GAA CCT CAT TAC T -3' (JU2 fwd; SEQ ID NO: 14),
- 5'- CAT AAC AGC AAT TCA CAT AAA CCT C -3' (JU3 fwd; SEQ ID NO: 15),
- 5'- GAC TGC GGA GGC TAA CT -3' (JU4 fwd; SEQ ID NO: 16),
- 5'- ATC CCT TTA TGA AGC GGC -3' (JU5 fwd; SEQ ID NO: 17), and
- 5'- TGA AAT GAA AGA CTG CGG AG -3' (MRSA direct RE2 fwd; SEQ ID NO: 92),

especially AR *mec* 11 fwd or AR *mec* 12 fwd or MRSA direct RE2 fwd, particularly MRSA direct RE2 fwd; and/or

(ii) a primer specific for MRSA type RE3,
particularly a primer comprising or consisting of a nucleic acid sequence selected from the group consisting of

- 5'- GCA AGG TAT AAT CCA ATA TTT CAT ATA TGT -3' (AR *mecA* 3/1; SEQ ID NO: 18),
- 5'- AGT TCC ATA ATC AAT ATA ATT TGT ACA GT -3' (AR *mecA* 3/2; SEQ ID NO: 19),
- 5'- ACA TCG TAT GAT ATT GCA AGG TA -3' (AR *mecA* 3/3; SEQ ID NO: 20),
- 5'- CTT TCA TTC TTT CTT GAT TCC ATT AG -3' (AR *mecA* 3/4; SEQ ID NO: 21),
- 5'- CAC TCT ATA AAC ATC GTA TGA TAT TGC -3' (AR *mecA* 3/5; SEQ ID NO: 22),
- 5'- TTC TTA ATT TAA TTG TAG TTC CAT AAT CAA -3' (AR *mecA 3/6;* SEQ ID NO: 23),
- 5'- AAT TAT ACA CAA CCT AAT TTT TAG TTT TAT -3' (AR *mecA 3/7;* SEQ ID NO: 24),
- 5'- AAT TTT TAG TTT TAT TTA TGA TAC GCT TC -3' (AR *mecA 3/8;* SEQ ID NO: 25),
- 5'- ACA CAA CCT AAT TTT TAG TTT TAT TTA TGA -3' (AR *mecA 3/9;* SEQ ID NO: 26),
- 5'- TTT ATT AAA CAC TCT ATA AAC ATC GTA TGA -3' (AR *mecA 3/10;* SEQ ID NO: 27),
- 5'- CCA CAT CTC ATT AAA TTT TTA AAT TAT ACA C -3' (AR *mecA* 3/13 SEQ ID NO: 28), and
- 5'- CCA CAT CTC ATT AAA TTT TTA AAT TAT ACA C -3' (MRSA direct RE3 fwd; SEQ ID NO: 93),

especially AR *mecA* 3/8 or AR *mecA* 3/1 or MRSA direct RE3 fwd, particularly MRSA direct RE3 fwd; and/or

(iii) a primer specific for MRSA type RE7,
particularly a primer comprising or consisting of a nucleic acid sequence selected from the group consisting of

- 5'- ATA TTA TAC ACA ATC CGT TTT TTA GTT TTA -3' (AR *mec* 5/1, SEQ ID NO: 29),
- 5'- ACA CAA TCC GTT TTT TAG TTT TAT TTA TG -3' (AR *mec* 5/2, SEQ ID NO: 30),
- 5'- TTC TAA TTT ATT TAA CAT AAA ATC AAT CCT -3' (AR *mec* 5/3, SEQ ID NO: 31),
- 5'- CAA TCC TTT TTA TAT TTA AAA TAT ATT ATA CAC -3' (AR *mec* 5/16 SEQ ID NO: 32), and
- 5'- CAA TCC TTT TTA TAT TTA AAA TAT ATT ATA CAC -3' (MRSA direct RE7 fwd; SEQ ID NO: 94),

especially AR *mec* 5/2 or AR *mec* 5/16 or MRSA direct RE7 fwd, particularly MRSA direct RE7 fwd.

The term "primer" is used herein as known to the expert skilled in the art and refers to "oligomeric compound" primarily to "oligonucleotides" but also to "modified oligonucleotides" that are able to "prime" DNA synthesis by a template-dependent DNA polymerase, i.e. the 3'-end of the e.g. oligonucleotide provides a free 3'-ooh group whereto further "nucleotides" may be attached by a template-dependent DNA polymerase establishing 3' to 5' phosphodiester linkage whereby desoxynucleoside triphosphates are used and whereby pyrophosphate is released. Therefore, there is - except for the intended function - no fundamental difference between a "primer", an "oligonucleotide" or a "probe" according to the invention.

In the invention, the set of primers consists of a primer specific for MRSA type RE2, a primer specific for MRSA type RE3 and a primer specific for MRSA type RE7, wherein the primers for MRSA type RE2, for MRSA type RE3 and for MRSA type RE7 consist of the sequences of SEQ ID NO: 92, SEQ ID NO: 93, and SEQ ID NO: 94, respectively. Further disclosed are sets wherein the primers for MRSA type RE2, for MRSA type RE3 and for MRSA type RE7 comprise or consist of the nucleic acid sequences of SEQ ID NO: 11, SEQ ID NO: 25, and SEQ ID NO: 30, respectively, or wherein the primers for MRSA type RE2, for MRSA type RE3 and for MRSA type RE7 comprise or consist of the nucleic acid sequences of SEQ ID NO: 12, SEQ ID NO: 28, and SEQ ID NO: 32, respectively.

In a preferred embodiment of the invention the set of primers additionally comprises a further primer specific for methicillin-resistant *S. aureus* (MRSA) as well as methicillin-sensitive *S. aureus* (MSSA) (primer for MRSA/MSSA). Examples of such a primer are selected from the group of primers comprising or consisting of the nucleic acid sequence selected from the group consisting of

- 5'- AGG AAA GAT GCT ATC TTC CGA -3' (AR *mec* 1 rev, SEQ ID NO: 33),
- 5'- GAA AGA TGC TAT CTT CCG AAG -3' (AR *mec* 2 rev, SEQ ID NO: 34),
- 5'- GAT GCT ATC TTC CGA AGG -3' (AR *mec* 3 rev, SEQ ID NO: 35),
- 5'- GTC ATT ACA TTA GAA ATA CAA GGA AAG AT -3' (AR *mec* 4 rev, SEQ ID NO: 36),
- 5'- GCC AAC GAA TAC TAG CC -3' (AR *mec* 5 rev, SEQ ID NO: 37),
- 5'- ACG AAT ACT AGC CAA AAT TAA ACC -3' (AR *mec* 6-2 rev, SEQ ID NO: 38),
- 5'- CAC AAT CCA CAG TCA TTA CAT TAG A -3' (AR *mec* 7 rev, SEQ ID NO: 39),
- 5'- CAA GGA AAG ATG CTA TCT TCC G -3' (JU1 rev, SEQ ID NO: 40),
- 5'- AGT CAT TAC ATT AGA AAT ACA AGG AAA GA -3' (JU2 rev, SEQ ID NO: 41),
- 5'- AGGAAAGATGCTATCTTCCGA -3' (JU3 rev, SEQ ID NO: 42),
- 5'- AGG AAA GAT GCT ATC TTC CGA -3' (JU4 rev, SEQ ID NO: 43),
- 5'- ACA ATC CAC AGT CAT TAC ATT AGA A -3' (JU5 rev, SEQ ID NO: 44),
  and
- 5'- CAA GGA AAG ATG CTA TCT TCC G -3' (MRSA direct rev; SEQ ID NO: 95),

especially AR *mec* 6-2 rev or JU1 rev or MRSA direct rev, particularly MRSA direct rev.

As detailed above, it is an object of the present invention to provide a highly reliable tool for the detection of MRSA. In order to reduce the susceptance to failure of the method of the invention, the number of primer used in the method may be restricted. Accordingly, in a further preferred embodiment of the invention the set of primers consists of at most 5, preferably at most 4 primers. Examples of such sets disclosed herein are :

- a primer specific for MRSA type RE2, a primer specific for MRSA type RE3, and a primer specific for MRSA type RE7, especially wherein one, two or three of the primers are selected from the nucleic acids of SEQ ID NO: 1 to 32 and 92 to 94,
- a primer specific for MRSA type RE2, a primer specific for MRSA type RE3, a primer specific for MRSA type RE7, and a primer specific for MRSA as well as MSSA (primer for MRSA/MSSA), especially wherein one, two, three or four of the primers are selected from the nucleic acids of SEQ ID NO: 1 to 44 and 92 to 95,
- AR *mec* 11 fwd, AR *mecA* 3/8 and AR *mec* 5/2 and optionally a reverse primer,
- AR *mec* 12 fwd, AR *mecA* 3/13 and AR *mec* 5/16 and optionally a reverse primer,
- AR *mec* 11 fwd, AR *mecA* 3/8, AR *mec 3/2* and AR *mec* 6-2 rev,
- AR *mec* 12 fwd, AR *mecA* 3/13, AR *mec* 5/16 and JU1 rev,
- MRSA direct RE2 fwd, MRSA direct RE3 fwd and MRSA direct RE7 fwd and optionally a reverse primer, or
- MRSA direct RE2 fwd, MRSA direct RE3 fwd, MRSA direct RE7 fwd and MRSA direct rev.

In another disclosed aspect, the primer for RE2, RE3, RE7 and/or MRSA/MSSA comprises or consists of a functionally active variant of any of the primers of SEQ ID NO: 1 to 44 or 92 to 95, optionally encompassed in any of the preferred combinations of particular primers as detailed above.

A functionally active variant of any of the primers of SEQ ID NO: I to 44 or 92 to 95 may be identified by using the primer in the method of the disclosure. A functionally active variant of a primer of any of the SEQ ID NO: 1 to 44 or 92 to 95 pertains to a primer which provides a similar or higher specificity and sensitivity in the method or kit of the disclosure as compared to the respective sequence of SEQ ID NO: 1 to 44 or 92 to 95.

The term "specificity" in a binary classification test with respect to a given class is the probability that the test correctly classifies cases not belonging to that class. That is, it is the proportion of true negatives of all negative cases in the population. It is a parameter of the test. In the present case, for the detection of MRSA to determine if a MRSA is present, the specificity to the presence of MRSA is the probability that if MRSA is absent, the test will be negative.

$$\text{specificity} = \frac{\text{number of true negatives}}{\text{number of true negatives} + \text{number of false positives}}$$

Accordingly, a specificity of 100 % means that the test recognizes all MRSA positive samples as MRSA positive.

A similar specificity in the context of the present invention relates to a specificity which is at most 10 %, preferably at most 5 %, more preferably at most 4, 3, 2 or 1 % smaller than that of the respective primer, if the primers (the variant and respective primer without mutation) are tested in the method of the invention, preferably the method as described in Example 3.

The term "sensitivity" in a binary classification test with respect to a given class is the probability that the test correctly classifies cases belonging to that class. That is, it is the proportion of true positives of all positives cases in the population. It is a parameter of the test. In the present case, for the detection of MRSA to determine if a MRSA is present, the sensitivity to the presence of MRSA is the probability that if MRSA is present, the test will be positive.

$$\text{sensitivity} = \frac{\text{number of true positives}}{\text{number of true positives} + \text{number of false negatives}}$$

A sensitivity of 100 % means that the test recognizes all sick people as such. We can always construct a trivial test that achieves 100 % sensitivity by classifying all test cases positive.

A similar sensitivity in the context of the present invention relates to a sensitivity which is at most 10 %, preferably at most 5 %, more preferably at most 4, 3, 2 or 1 % smaller than that of the respective primer, if the primers (the variant and respective primer without mutation) are tested in the method of the invention, preferably the method as described in Example 3.

In a preferred aspect the primer comprises or consists of a functionally active variant of any of the primers of SEQ ID NO: 1 to 44 or 92 to 95, wherein

(a) the functionally active variant is a functionally active part of a primer of SEQ ID NO: 1 to 44 or 92 to 95, the part encompassing at least 70 %, preferably at least 80 %, more preferably at least 90 %, most preferably at least 95 % of any of the sequences of SEQ ID NO: 1 to 44 or 92 to 95;

(b) the functionally active variant is a functionally active variant having at least 70 %, preferably at least 80 %, more preferably at least 90 %, most preferably at least 95 % sequence identity with any of the sequences of SEQ ID NO: 1 to 44 or 92 to 95;

(c) the functionally active variant is a probe of SEQ ID NO: 1 to 44 or 92 to 95 comprising at least one chemically modified nucleic acid; and/or

(d) the functionally active variant is a functionally active variant complementary to any of the sequences of SEQ ID NO: 1 to 44 or 92 to 95 or a functionally active variant of (a), (b) and/or (c).

The part of the primer may be obtained by terminal deletion of nucleotides (at the 5' end and/or the 3' end). Sequence identity may be determined by sequence alignment. Methods of alignment of sequences for comparison are well known in the art. Various programs and alignment algorithms have been described e.g. in Smith and Waterman, Adv. Appl. Math. 2: 482, 1981 or Pearson and Lipman, Proc. Natl. Acad. Sci. U.S.A. 85: 2444-2448, 1988.

The NCBI Basic Local Alignment Search Tool (BLAST) (Altschul et al., J. Mol. Biol. 215: 403-410, 1990) is available from several sources, including the National Center for Biotechnology Information (NCBI, Bethesda, MD) and on the Internet, for use in connection with the sequence analysis programs blastp, blastn, blastx, tblastn and tblastx. Variants of a primer of any of the sequences of SEQ ID NO: 1 to 44 or 92 to 95 are typically characterized using the NCBI Blast 2.x.

The variant may e.g. vary from the sequence of SEQ ID NO: 1 to 44 or 92 to 95 by one or more nucleotide additions, deletions or substitutions, particularly one or more nucleotide additions, deletions or substitutions at the 5' end and/or the 3' end of the respective sequence of SEQ ID NO: 1 to 44 or 92 to 95.

As detailed above, the primer (and/or the probe) may be chemically modified, i.e. the primer and/ or the probe comprise a modified nucleotide or a non-nucleotide compound. The probe (or the primer) is then a modified oligonucleotide. "Modified nucleotides" (or "nucleotide analogs") differ from a natural "nucleotide" by some modification but still consist of a base, a pentofuranosyl sugar, a phosphate portion, base-like, pentofuranosyl sugar-like and phosphate-like portion or combinations thereof. For example, a "label" may be attached to the base portion of a "nucleotide" whereby a "modified nucleotide" is obtained. A natural base in a "nucleotide" may also be replaced by e.g. a 7-desazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

A "modified oligonucleotide" (or "oligonucleotide analog") belongs to another specific subgroup of the "oligomeric compounds", that possesses one or more "nucleotides", one or more "non-nucleotide compounds" or "modified nucleotides" as "monomeric units". Thus, the terms "modified oligonucleotide" (or "oligonucleotide analog") refers to structures that function in a manner substantially similar to "oligonucleotides" and are used interchangeably throughout the application. From a synthetical point of view, a "modified oligonucleotide" (or a "oligonucleotide analog") can be for example made by chemical modification of "oligonucleotides" by appropriate modification of the phosphate backbone, ribose unit or the nucleotide bases (Uhlmann and Peyman, Chemical Reviews 90 (1990) 543; Verma S., and Eckstein F., Annu. Rev. Biochem. 67 (1998) 99-134). Representative modifications include phosphorothioate, phosphorodithioate, methyl phosphonate, phosphotriester or phosphoramidate inter-nucleoside linkages in place of phosphodiester inter-nucleoside linkages; deaza or aza purines and pyrimidines in place of natural purine and pyrimidine bases, pyrimidine bases having substituent groups at the 5 or 6 position; purine bases having altered substituent groups at the 2, 6 or 8 positions or 7 position as 7-deazapurines; sugars having substituent groups at, for example, their 2' position; or carbocyclic or acyclic sugar analogs. Other modifications are known to those skilled in the art. Such "modified oligonucleotides" (or "oligonucleotide analogs") are best described as being functionally interchangeable with, yet structurally different from, natural "oligonucleotides" (or synthetic "oligonucleotides" along natural lines). In more detail, exemplary modifications are disclosed in Verma S., and Eckstein F., Annu. Rev. Biochem. 67 (1998) 99-134 or WO 02/12263. In addition, modification can be made wherein nucleoside units are joined through groups that substitute for the internucleoside phosphate or sugar phosphate linkages. Such linkages include those disclosed in Verma S., and Eckstein F., Annu. Rev. Biochem. 67 (1998) 99-134. When other than phosphate linkages are utilized to link the nucleoside units, such structures have also been described as "oligonucleosides".

However, alternative primers that amplify a nucleic acid molecule encoding MRSA, e.g., nucleic acids encoding alternative portions of portions of RE2, RE3 or RE7, can be designed using, for example, a computer program such as OLIGO (Molecular Biology Insights Inc., Cascade, CO). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 8 to 50 nucleotides in length (e.g. 8, 10, 12, 14, 16, 18, 20, 22, 24, 26, 28, 30, 32, 34, 36, 38, 40, 42, 44, 46, 48, or 50 nucleotides in length).

The sequences of SCC*mec* of various subtypes of MRSA are available to public, e.g. at the nucleotide data base of NCBI (National Center for Biotechnology Information) (see e.g. AB033763 for type-I staphylococccal cassette chromosome *mec*: strain NCTC10442; D86934 for type-II staphylococcal cassette chromosome *mec*: strain N315; AB047089, right extremity of type-III Staphylococcal cassette chromosome *mec* and its flanking chromosomal region: strain 85/3907; AB063172 for type-IV.1 (IVa) staphylococcal cassette chromosome *mec*: strain CA05(JCSC1968); or AB121219 type-V staphylococcal cassette chromosome *mec*: strain JCSC3624(WIS)).

"Primers specific for RE2" or "primers specific for RE3" or "primers specific for RE7" as used herein refer to oligonucleotide primers that anneal to nucleic acid sequences encoding RE2 or RE3 or RE7, respectively, and initiate synthesis therefrom under appropriate conditions.

In addition to the set of primers as defined in appended claim 1, the method of the invention uses a pair of probes in order to detect the presence or absence of MRSA. The term "probe" refers to synthetically or biologically produced nucleic acids (DNA or RNA) which, by design or selection, contain specific nucleotide sequences that allow them to hybridize under defined predetermined stringencies specifically (i.e., preferentially) to "target nucleic acids", in the present case to a MRSA (target) nucleic acid. A "probe" can be identified as a "detection probe" meaning that it detects the target nucleic acid.

In a preferred aspect of the disclosure at least one probe of the pair of probes comprises or consists of a fluorescent moiety and a nucleic acid sequences selected from the group consisting of

- 5'- AAG TCG CTT TGC CTT TGG GTC A -3' (AR *mec* Fluo 1 w/o label; SEQ ID NO: 45),
- 5'- TAC AAA GTC GCT TTG CCT TTG GGT CA -3' (**AR** *mec* Fluo 2 w/o label; SEQ ID NO: 46),

- 5'- GGC CGT TTG ATC CGC CAA T -3' (AR *mec* Fluo 3 w/o label; SEQ ID NO: 47),
- 5'- AAG TCG CTT TGC CCT TGG GTA -3' (AR *mec* Fluo 4 w/o label; SEQ ID NO: 48),
- 5'- AAG TCG CTT TGC CCT TGG GT -3' (AR *mec* Fluo 4-2 w/o label; SEQ ID NO: 49),
- 5'- AAG TCG CTT TGC CCT TGG GTC A -3' (AR *mec* Fluo 4-3 w/o label; SEQ ID NO: 50),
- 5'- AAG TCG CTT TGC CCT TGG G -3' (AR *mec* Fluo 4-GV w/o label; SEQ ID NO: 51),
- 5'-CAA GAA TTG AAC CAA CGC AT -3' (AR *mec* Fluo 4k w/o label; SEQ ID NO: 52),
- 5'- CAA TGA CGA ATA CAT AGT CGC TTT GCC CTT -3' (AR *mec* Fluo 5 w/o label; SEQ ID NO: 53),
- 5'- CGT TTG ATC CGC CAA TGA CGA -3' (AR *mec* Fluo 6 w/o label; SEQ ID NO: 54),
- 5'- GCC AAT CCT TCG GAA GAT AGC A -3' (AR *mec* Fluo 7 w/o label; SEQ ID NO: 55),
- 5'- ATT AAC ACA ACC CGC ATC -3' (AR *mec* Fluo UR w/o label; SEQ ID NO: 56),
- 5'- GTC GCT TTG CCC TTG GGT C -3' JU1 probe 1 w/o label; SEQ ID NO: 57),
- 5'- TCG CTT TGC CCT TGG GTC AT -3' JU2 probe 1 w/o label; SEQ ID NO: 58),
- 5'- GGC CGT TTG ATC CGC CAA T -3' JU3 probe 1 w/o label; SEQ ID NO: 59),
- 5'- GTC CTT GTG CAG GCC GTT TGA T -3' JU4 probe 1 w/o label; SEQ ID NO: 60),
- 5'- CTT GGG TCA TGC GTT GGT TCA ATT -3' JU5 probe1 w/o label; SEQ ID NO: 61),
- 5'- CGA ATA CAA AGT CGC TTT GCC CTT GGG -3' (AR *mec* 640 3 w/o label; SEQ ID NO: 62),
- 5'- ATG CGT TGG TTC AAT TCT TG -3' (AR *mec* 640 4 w/o label; SEQ ID NO: 63),
- 5'- GCG TTG GTT CAA TTC TTG GG -3' (AR *mec* 610 4-3 w/o label; SEQ ID NO: 64),
- 5'- ACC CAA GGG CAA AGC GAC TT -3' (AR *mec* 640 4k w/o label; SEQ ID NO: 65),
- 5'- GGT AAT GCG TTG GTT CAA TTC TTG -3' (AR *mec* 640 5 w/o label; SEQ ID NO: 66),
- 5'- ACA AAG TCG CTA TGC CCT TGG GTC A -3' (AR *mec* 640 6 w/o label; SEQ ID NO: 67),
- 5'- CTT TCC TTG TAT TTC TAA TGT AAT GAC TG -3' (AR *mec* 640 7 w/o label; SEQ ID NO: 68),
- 5'- TTG ATG TGG GAA TGT CAT TTT GCT GAA -3' (AR *mec* 640 UR w/o label; SEQ ID NO: 69),
- 5'- GCG TTG GTT CAA TTC TTG GGC CAA T -3' JU1 probe 2 w/o label; SEQ ID NO: 70),
- 5'- GTT GGT TCA ATT CTT GGG CCA ATC CTT CG -3' JU2 probe 2 w/o label; SEQ ID NO: 71),
- 5'- CGA ATA CAA AGT CGC TTT GCC CTT GG -3' JU3 probe 2 w/o label; SEQ ID NO: 72),
- 5'- GCC AAT GAC GAA TAC AAA GTC GCT TTG CC -3' JU4 probe2 w/o label; SEQ ID NO: 73),
- 5'- TGG GCC AAT CCT TCG GAA GAT AGC A -3' JU5 probe2 w/o label; SEQ ID NO: 74),
- 5'- ATG CGT TGG TTC GAT TCT TG -3' (AR *mec* 610 4-MM2 w/o label; SEQ ID NO: 75),
- 5'- CAT GCG TTG GTT CGA TTC TTG -3' (AR *mec* 610 4-MM2-GV w/o label; SEQ ID NO: 76),
- 5'- AAG TCG CTT TGC CCT TGG G -3' (MRSA direct Fluos w/o label; SEQ ID NO: 96), and
- 5'- CAT GCG TTG GTT CGA TTC TTG -3' (MRSA direct Red 610 w/o label; SEQ ID NO: 97),

especially AR *mec* Fluo 4 w/o label and/or AR *mec* 610 4-MM2 w/o label or especially AR *mec* Fluo 4-GV w/o label and/or AR *mec* 610 4-MM2-GV w/o label or especially MRSA direct Fluos w/o label and/or MRSA direct Red 610 w/o label, preferably MRSA direct Fluos w/o label and/or MRSA direct Red 610 w/o label, more preferably MRSA direct Fluos w/o label and MRSA direct Red 610 w/o label.

In an embodiment of the invention, the first probe comprises or consists of a fluorescent moiety and the nucleic acid sequences of SEQ ID NO: 96 and the second probe comprises or consists of a fluorescent moiety and a nucleic acid sequences of SEQ ID NO: 97 or vice versa. According to the invention the first MRSA probe of the pair of MRSA probes is labeled with a donor fluorescent moiety and the second MRSA probe of the pair of MRSA probes is labeled with a corresponding acceptor fluorescent moiety. Examples of suitable and preferred labels are listed below, wherein each of the probes may be labeled with any of the labels. However, a preferred pair of probes is one, wherein the first probe including the first label is fluorescein-5'- AAG TCG CTT TGC CCT TGG GTA -3' (AR *mec* Fluo 4) and wherein the second probe including the second label is LC-Red 610-5'- ATG CGT TGG TTC GAT TCT TG -3' (AR *mec* 610 4-MM2). An alternative preferred pair of probes is one, wherein the first probe including the first label is fluorescein-5'-AAG TCG CTT TGC CCT TGG G -3' (AR *mec* Fluo 4-GV) and wherein the second probe including the second label is LC-Red 610-5'- CAT GCG TTG GTT CGA TTC TTG -3' (AR *mec* 610 4-MM2-GV). An even more preferred pair of probes is one, wherein the first probe including the first label is 5'- AAG TCG CTT TGC CCT TGG G -3'-fluorescein (MRSA direct Fluos) and wherein the second probe including the second labels is LC-Red 610-5'-CAT GCG TTG GTT CGA TTC TTG -3' (MRSA direct Red 610).

In another aspect the probe comprises or consists of a fluorescent moiety and a functionally active variant of any of the probes of SEQ ID NO: 45 to 76, 96 or 97, particularly wherein

(a) the functionally active variant is a functionally active part of a probe of any of the sequences of SEQ ID NO: 45 to 76, 96 or 97, the part encompassing at least 70 %, preferably at least 80 %, more preferably at least 90 %, most preferably at least 95 % of any of the sequences of SEQ ID NO: 45 to 76, 96 or 97;

(b) the functionally active variant is a functionally active variant having at least 70 %, preferably at least 80 %, more preferably at least 90 %, most preferably at least 95 % sequence identity with any of the sequences of SEQ ID NO: 45 to 76, 96 or 97;

(c) the functionally active variant is a probe of SEQ ID NO: 45 to 76, 96 or 97 comprising at least one chemically modified nucleic acid; and/or

(d) the functionally active variant is a functionally active variant complementary to any of the sequences of SEQ ID NO: 45 to 76, 96 or 97 or a functionally active variant of (a), (b) and/or (c).

A functionally active variant of any of these probes may be identified by using the variant and the respective probe in the method of the invention. A functionally active variant of a probe comprising any of the SEQ ID NO: 45 to 76, 96 or 97 pertains to a probe which has a similar or higher specificity and sensitivity as the respective sequence of SEQ ID NO: 45 to 76, 96 or 97. The terms "specificity" and "sensitivity" are as defined above for the primers and may be determined as detailed for the variants of primers.

The part of the probe may be obtained by terminal deletion of nucleotides (at the 5' end and/or the 3' end). Sequence identity may be determined by sequence alignment. Methods of alignment of sequences for comparison are well known in the art. Various programs and alignment algorithms have been described e.g. in Smith and Waterman, Adv. Appl. Math. 2: 482, 1981 or Pearson and Lipman, Proc. Natl. Acad. Sci. U.S.A. 85: 2444-2448, 1988.

The NCBI Basic Local Alignment Search Tool (BLAST) (Altschul et al., J. Mol. Biol. 215: 403-410, 1990) is available from several sources, including the National Center for Biotechnology Information (NCBI, Bethesda, MD) and on the Internet, for use in connection with the sequence analysis programs blastp, blastn, blastx, tblastn and tblastx. Variants of a probe comprising any of the sequences of SEQ ID NO: 45 to 76, 96 or 97 are typically characterized using the NCBI Blast 2.x.

The variant may e.g. vary from the sequence of SEQ ID NO: 45 to 76, 96 or 97 by one or more nucleotide additions, deletions or substitutions, particularly one or more nucleotide additions, deletions or substitutions at the 5' end and/or the 3' end of the respective sequence of SEQ ID NO: 45 to 76, 96 or 97.

Additionally, it is referred to the above details regarding variants of primers, which may be applied analogously to the variants of probes.

In an aspect of the disclosure the set of primers comprises or consists of

a) AR *mec* 11 fwd, AR *mecA* 3/8, AR *mec* 5/2 and optionally AR *mec* 6-2 rev and wherein the first probe is fluorescein-5'- AAG TCG CTT TGC CCT TGG GTA -3' (AR *mec* Fluo 4) and wherein the second probe is LC-Red 610-5'- ATG CGT TGG TTC GAT TCT TG -3' (AR *mec* 610 4-MM2) or

b) AR *mec* 12 fwd, AR *mecA* 3/13, AR *mec* 5/16 and optionally JU1 rev and wherein the first probe is fluorescein-5'- AAG TCG CTT TGC CCT TGG G -3' (AR *mec* Fluo 4-GV) and wherein the second probe is LC-Red 610-5'- CAT GCG TTG GTT CGA TTC TTG -3' (AR *mec* 610 4-MM2-GV) or

c) MRSA direct RE2 fwd, MRSA direct RE3 fwd, MRSA direct RE7 fwd and optionally MRSA direct rev and wherein first label is 5'- AAG TCG CTT TGC CCT TGG G -3'-fluorescein (MRSA direct Fluos) and wherein the second probe is LC-Red 610-5'- CAT GCG TTG GTT CGA TTC TTG -3' (MRSA direct Red 610).

Designing oligonucleotides to be used as (hybridization) probes can be performed in a manner similar to the design of primers, although the members of a pair of probes preferably anneal to an amplification product within no more than 5 nucleotides of each other on the same strand such that fluorescent resonance energy transfer (FRET) can occur (e.g., within no more than 1, 2, 3, or 4 nucleotides of each other). This minimal degree of separation typically brings the respective fluorescent moieties into sufficient proximity such that FRET occurs (see below). It is to be understood, however, that other separation distances (e.g., 6 or more nucleotides) are possible provided the fluorescent moieties are appropriately positioned relative to each other (for example, with a linker arm) such that FRET can occur. In addition, probes can be designed to hybridize to targets that contain a mutation or polymorphism, thereby allowing differential detection of MRSA based on either absolute hybridization of different pairs of probes corresponding to each particular MRSA subtype to be distinguished or differential melting temperatures between, for example, members of a pair of probes and each amplification product generated from a MRSA. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are 8 to 50 nucleotides in length (e.g. 8, 10, 12, 14, 16, 18, 20, 22, 24, 26, 28, 30, 32, 34, 36, 38, 40, 42, 44, 46, 48, or 50 nucleotides in length).

The invention provides methods for detecting the presence or absence of MRSA in a sample. Methods provided by the invention avoid problems of sample contamination, false negatives and false positives. The methods include performing at least one amplifying step and at least one hybridizing step. An amplification step includes contacting the sample with a set of suitable primers to produce an amplification product if a MRSA nucleic acid molecule is present in the sample. Each of the primers anneals to a target within the MRSA target nucleic acid molecule such that at least a portion of the amplification product contains nucleic acid sequence corresponding to the respective MRSA nucleic acid, wherein the set of primers is selected to enable detection of MRSA types I to V. The hybridizing step includes contacting the sample with a pair of MRSA probes. Generally, the members of the pair of MRSA probes hybridize to the appropriate amplification product within no more than five nucleotides of each other. According to the invention, a first MRSA probe of the pair of MRSA probes is labeled with a donor fluorescent moiety and a second MRSA probe of the pair of MRSA probes is labeled with a corresponding acceptor fluorescent moiety. The method further includes detecting the presence or absence of FRET between the donor fluorescent moiety of the first MRSA probe and the corresponding acceptor fluorescent moiety of the second MRSA probe. Multiple steps of amplification and hybridisation can be performed, preferably in a thermocycler.

As used herein, "amplifying" refers to the process of synthesizing nucleic acid molecules that are complementary to one or both strands of a template nucleic acid (e.g., MRSA nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. The denaturing, annealing and elongating steps each can be performed once. Generally, however, the denaturing, annealing and elongating steps are performed multiple times such that the amount of amplification product is increasing, oftentimes exponentially, although exponential amplification is not required by the present methods. Amplification typically requires the presence of deoxyribonucleoside triphosphates, a DNA polymerase enzyme (e.g. Taq Polymerase) and an appropriate buffer and/or co-factors for optimal activity of the polymerase enzyme (e.g., $MgCl_2$ and/or KCl).

If amplification of MRSA nucleic acid occurs and an amplification product is produced, the step of hybridizing results in a detectable signal based upon FRET between the members of the pair of probes. As used herein, "hybridizing" refers to the annealing of probes to an amplification product. Hybridization conditions typically include a temperature that is below the melting temperature of the probes but that avoids nonspecific hybridization of the probes.

In the method of the invention the presence or absence of MRSA is detected using Fluorescence Resonance Energy Transfer (FRET) (see, for example, U.S. Patent Nos. 4,996,143, 5,565,322, 5,849,489, and 6,162,603). FRET is a technique for measuring interactions between two molecules, in the present case two probes. In this technique, two different fluorescent molecules (fluorophores or labels) are genetically fused to a pair of probes suitable for the detection of MRSA.

Resonance energy transfer is a mechanism by which energy is transferred directly from one molecule to another. The principle of FRET is based on the combined characteristics of the two labels. If a label is excited with a light of a particular wavelength (absorption frequency) its re-emits that energy at a different wavelength (the emission frequency). In FRET the first label is excited with which in turn emits light having the emission frequency. If the emission peak of the first label (donor) overlaps with the excitation peak of the second label (acceptor), proximity of the two labels can be determined, since the first label transfers energy to the second label and the second label emits light at its own emission frequency. The net result is that the donor emits less energy than it normally would (since some of the energy it would radiate as light gets transferred to the acceptor instead), while the acceptor emits more light energy at its excitation frequency (because it is getting extra energy input from the donor fluorophore).

The benefit of FRET technology is that it has an excellent resolution. The physics of the FRET energy transfer between donor and acceptor (which is non-radiactive) is such that the efficiency falls off with the sixth power of the distance between molecules. Thus, FRET in general occurs when the two fluorophores are within 20 to 100 Á (0.002 to 0.01 $\mu$m) of each other, which means that the fluorophores must be brought together quite closely.

As used herein, two oligonucleotide probes, each containing a fluorescent moiety, can hybridize to an amplification product at particular positions determined by the complementarity of the oligonucleotide probes to the target nucleic acid sequence, i.e. the MRSA nucleic acid. Upon hybridization of the oligonucleotide probes to the amplification product at the appropriate positions, a FRET signal is generated. In a preferred embodiment the members of the pair of MRSA probes (detection probes) hybridize to the amplification product within no more than five, four or three nucleotides, particularly within no more than two nucleotides of each other, especially within no more than one nucleotide of each other.

As detailed above, each probe of the pair of probes is labeled with a suitable label/fluorophore/fluorescent moiety. "Labels", often referred to as "reporter groups", "fluorophore" or "fluorescent moiety", are generally groups that mark or label a target nucleic acid to make it distinguishable from the remainder (nucleic acids having attached a "label" can also be termed labeled nucleic acid binding compounds, labeled probes or just probes).

As used herein, "fluorescence resonance energy transfer relationship" and similar terms refer to adjacent hybridization of an "probe" labeled with a "donor fluorescent moiety" and another "probe" labeled with an "acceptor fluorescent moiety" to a "target nucleic acid" such that the "donor fluorescent label" can transfer resonance energy to the "acceptor fluorescent label" such that the "acceptor fluorescent label" produces a measurable fluorescence emission. If the "donor fluorescent label" and "acceptor fluorescent label" are spaced apart by too great a distance, then the "donor fluorescent label" cannot transfer resonance energy to the "acceptor fluorescent label" such that the "acceptor fluorescent label" emits measurable fluorescence, and hence the "donor fluorescent label" and "acceptor fluorescent label" are not in resonance energy transfer relationship.

Suitable labels are known in the art and the skilled practitioner is able to choose a suitable combination of labels for both probes. As used herein with respect to donor and corresponding acceptor fluorescent moieties, "corresponding" refers to an acceptor fluorescent moiety having an emission spectrum that overlaps the excitation spectrum of the donor fluorescent moiety. However, both signals should be separable from each other. Accordingly, the wavelength maximum of the emission spectrum of the acceptor fluorescent moiety preferably should be at least 30 nm, more preferably at least 50 nm such as at least 80 nm, at least 100 nm or at least 140 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Förster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (> 600 nm). However, the main target is to obtain signals separable from each other.

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC -Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO).

Preferred labels according to the invention are fluorescent labels, which are e.g. fluorescent dyes as a fluorescein dye, a rhodamine dye, a cyanine dye, and a coumarin dye. For example, the donor fluorescent moiety may be fluorescein and/or the acceptor fluorescent moiety may be selected from the group consisting of LC-Red 610, LC-Red 640, LC-Red 670, LC-Red 705, Cy5, and Cy5.5, preferably LC-Red 610 or LC-Red 640. More preferably the donor fluorescent moiety is fluorescein and the acceptor fluorescent moiety is LC-Red 640 or LC-Red 610.

For the donor-acceptor pair of fluorescein-rhodamine one might use a 470-490 nm excitation filter, and a 500-520 nm emission filter for collecting the light from the fluorescein donor. One might then use a 600-650 nm emission filter for collecting the light from the rhodamine acceptor. These emission filters need to collect just the shorter wavelength range of the donor, and just the long emission tail of the acceptor, to avoid cross-talk between the two image channels. That is, one would want to avoid collecting fluorescein emission in the rhodamine channel and vica versa. The problem is that for FRET to work, the donor emission and acceptor excitation spectra must overlap (high overlap is good), but for good signal-to-noise ratio imaging one must avoid collecting the "wrong" photons through a filter.

The donor and acceptor fluorescent moieties can be attached to the appropriate probe oligonucleotide via a linker arm. The length of each linker arm can be important, as the linker arms will affect the distance between the donor and the acceptor fluorescent moieties. The length of a linker arm for the purpose of the present invention is the distance in Ångstroms from the nucleotide base to the fluorescent moiety. In general, a linker arm is from about 10 to about 25 Å. The linker arm may be of the kind described in WO 84/03285. WO 84/03285 also discloses methods for attaching linker arms to particular nucleotide bases, and also for attaching fluorescent moieties to a linker arm.

An acceptor fluorescent moiety such as an LC-Red 640(-NHS-ester), LC-Red 610(-NHS-ester) or LC-Red 670 (-NHS-ester) can be combined with C6-Phosphoramidites (available from ABI (Foster City, CA) or Glen Research (Sterling, VA)) to produce, for example, LC-Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, MA)), amide-linkers (fluorescein-NHS-ester-derived, such as fluorescein-CPG from BioGenex (San Ramon, CA)), or 3'-amino-CPG's that require coupling of a fluorescein-NHS-ester after oligonucleotide synthesis.

In one embodiment of the invention the detecting step comprises exciting the sample at a wavelength absorbed by the donor fluorescent moiety and visualizing and/or measuring the wavelength emitted by the acceptor fluorescent moiety. Preferably, the detecting comprises quantitating the FRET. Fluorescent analysis can be carried out using, for example, a photon counting epifluorescent microscope system (containing the appropriate dichroic mirror and filters for monitoring fluorescent emission at the particular range), a photon counting photomultiplier system or a fluorometer.

Excitation to initiate energy transfer can be carried out with an argon ion laser, a high intensity mercury (Hg) arc lamp, a fiber optic light source, or other high intensity light source appropriately filtered for excitation in the desired range.

Another subject of the invention relates to an alternative method of detecting the presence or absence of methicillin-resistant *S. aureus* (MRSA) in a sample, the method comprising

(a) performing an amplifying step comprising contacting the sample with a set of MRSA primers to produce an amplification product if MRSA is present in the sample,
(b) performing a hybridizing step comprising contacting the amplification product of step (a) with a MRSA probe, wherein the MRSA probe is labeled with a donor fluorescent moiety and with a corresponding acceptor fluorescent moiety; and
(c) detecting the presence or absence of fluorescence resonance energy transfer (FRET) between the donor fluorescent moiety and the acceptor fluorescent moiety of the MRSA probe, wherein the absence of FRET is indicative of the presence of MRSA in the sample and wherein the presence of FRET is indicative of the absence of MRSA in the sample,

wherein the method is capable of detecting each of Staphylococcal Chromosomal Cassettes (SCC*mec*) types I to V of MRSA, and wherein the set of primers is as defined in appended claims 1-6.

A common format of FRET technology utilizes two hybridization probes, wherein each probe can be labeled with a different fluorescent moiety and are generally designed to hybridize in close proximity to each other in a target DNA molecule (*e.g.*, an amplification product) (see above method described first). However, an alternative FRET format utilizes hydrolyzation probes (second method of the invention) to detect the presence or absence of an amplification product, and hence, the presence or absence of MRSA. This technology utilizes one single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hydrolyzation probe binds to the target DNA (*i.e.,* the amplification product) and is degraded by the 5' to 3' exonuclease activity of the Taq Polymerase during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM® 7700 Sequence Detection System (Applied Biosystems, Foster City, CA) uses hydrolyzation probe technology, and is suitable for performing the methods described herein for detecting MRSA. Information on PCR amplification and detection using an ABI PRISM® 770 system can be found at http://www.appliedbiosystems.com/products.

Examples of suitable hydrolyzation probes are:

TP mec 1: 5'- AAG TCG CTT TGC CCT TGG GTC AT -3' (SEQ ID NO: 87) and
TP mec 2: 5'- TGC TCA ATT AAC ACA ACC CGC ATC A -3'(SEQ ID NO: 88).

In a preferred embodiment of the invention the method may be further defined by one or more of the following features:

a) the set of primers is as defined above in the context of the first method of the invention, comprising or consisting of a primer specific for MRSA type RE2, a primer specific for MRSA type RE3 and a primer specific for MRSA type RE7
b) the probe comprises or consists of two fluorescent moieties, preferably as defined in above in the context of the first method of the invention, and a nucleic acid sequence of any of the SEQ ID NOS: 45 to 76, 96 or 97 or a functionally active variant thereof as defined in the context of the first method of the invention
c) the amplifying step employs a polymerase enzyme having 5' to 3' exonuclease activity
d) the donor and acceptor fluorescent moieties are within no more than 5 nucleotides of each other on the probe
e) the probe comprises a nucleic acid sequence that permits secondary structure formation, wherein the secondary structure formation results in spatial proximity between the donor and acceptor fluorescent moiety
f) the acceptor fluorescent moiety is a quencher.

Molecular beacons in conjunction with FRET also can be used to detect the presence of an amplification product using the real-time PCR methods of the invention (alternative second method of the invention). Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (e.g., a hairpin). As a result of secondary structure formation within the probe, both fluorescent moieties are in spatial proximity when the probe is in solution. After hybridization to the target nucleic acids (i.e., amplification products), the secondary structure of the probe is disrupted and the fluorescent moieties become separated from one another such that after excitation with light of a suitable wavelength, the emission of the first fluorescent moiety can be detected.

Examples of suitable molecular beacons are:

MB mec 1: 5'- GCC GCG CTG CTC AAT TAA CAC AAC CCG CGC GGC-3' (SEQ ID NO: 89) and
MB mec 2: 5'- GCC GCG CAT GCG TTG GTT CAA TTC TGC GCG GC-3' (SEQ ID NO: 90).

It is noted that the above details on the first method of the invention apply analogously to the second method of the invention, with the exception that in the second method of the invention only one probe labeled with a donor fluorescent moiety and with a corresponding acceptor fluorescent moiety is used.

Accordingly, as used herein, "fluorescence resonance energy transfer relationship" and similar terms refer to a "probe" labeled with a "donor fluorescent moiety" and an "acceptor fluorescent moiety" such that the "donor fluorescent label" can transfer resonance energy to the "acceptor fluorescent label" such that the "acceptor fluorescent label" produces a measurable fluorescence emission. If the "donor fluorescent label" and "acceptor fluorescent label" are spaced apart by too great a distance, then the "donor fluorescent label" cannot transfer resonance energy to the "acceptor fluorescent label" such that the "acceptor fluorescent label" emits measurable fluorescence, and hence the "donor fluorescent label" and "acceptor fluorescent label" are not in resonance energy transfer relationship. Preferably, the acceptor fluorescent moiety is a quencher absorbing the energy emitted by the donor fluorescent moiety.

The first (using (a) hybridization probe(s)) and second method (using (a) hydrolyzation probe(s) or (a) molecular beacon(s)) according to the invention may be performed in a format for the use in the LightCycler® instrument which is described in US 6,174,670. This format comprises amplification and detection whereby the latter uses the detection of the fluorescence for the detection of the binding product between a pair of probes or a single probe and the target nucleic acid. These formats apply the FRET technology (see, for example, US Patent Nos. 4,996,143, 5,565,322, 5,849,489, and 6,162,603). As used herein, two probes, each containing a fluorescent label, or a single probe containing both fluorescent labels can hybridize to an amplification product at particular positions determined by the complementarity of the probes to the target nucleic acid. The fluorescent label may be a donor or acceptor fluorescent label. Upon hybridization of the probe(s) to the amplification product at the appropriate positions, a FRET signal is generated. The exemplified components as detailed above may be used (e.g. the detection devices, light sources etc.)

In a preferred embodiment of the invention the amplifying step a) in the first method according to the invention comprises contacting the sample with the set of primers and optionally a suitable polymerase, especially by PCR, to produce an amplification product if MRSA nucleic acid is present in said sample, wherein said hybridizing step b) comprises contacting said sample with the pair of probes, wherein the members of said pair of probes hybridize to said amplification product within no more than five nucleotides of each other, wherein the first probe of said pair of probes is labeled with a donor fluorescent label and wherein the second probe of said pair of probes is labeled with a corresponding acceptor fluorescent label; and detecting the binding product between the MRSA nucleic acid and the pair of probes in step c) by detecting the presence or absence of FRET between said donor fluorescent label of said first probe and said acceptor fluorescent label of said second probe, wherein the presence of FRET is indicative of the presence of the target nucleic acid in the sample, and wherein the absence of FRET is indicative of the absence of the target nucleic acid in the sample.

In an alternative preferred embodiment of the invention the amplifying step a) in the second method according to the invention comprises contacting the sample with the set of primers and optionally a suitable polymerase, especially by PCR, to produce an amplification product if MRSA nucleic acid is present in said sample, wherein said hybridizing step b) comprises contacting said sample with the probe, wherein the probe is labeled with a donor fluorescent label and a corresponding acceptor fluorescent label, e.g. a quencher, and wherein the probe hybridizes to said amplification product; and detecting the binding product between the MRSA nucleic acid and the probe in step c) by detecting the presence or absence of FRET between said donor fluorescent label of the probe and the acceptor fluorescent label, wherein the presence or absence of FRET is indicative of the presence or absence of the target nucleic acid in the sample.

U.S. Patent Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in the present invention include oligonucleotides capable of acting as a point of initiation of nucleic acid synthesis within a MRSA nucleic acid sequence.

A primer can be purified from a restriction digest by conventional methods, or it can be produced synthetically. A primer is preferably single-stranded for maximum efficiency in amplification. The primer may be produced by chemical synthesis.

PCR assays can employ nucleic acid (DNA or RNA) template in general DNA. The template nucleic acid need not be purified; it may be a minor fraction of a complex mixture, such as MRSA toxin nucleic acid contained in human cells. DNA (or RNA) may be extracted from any sample such as body fluids or swabs by routine techniques such as those described in Diagnostic Molecular Microbiology: Principles and Applications (Persing et al. (eds), 1993, American Society for Microbiology, Washington D.C).

The oligonucleotide primers are combined with other PCR reagents under reaction conditions that induce primer extension. For example, chain extension reactions generally include 50 mM KCl, 10 mM Tris-HCl (pH 8.3), 1.5 mM $MgCl_2$, 0.001% (w/v) gelatin, 0.5-1.0 μg denatured template DNA, 50 pmoles of each oligonucleotide primer, 2.5 U of Taq polymerase, and 10 % DMSO. The reactions usually contain 150 to 320 μM each of dATP, dCTP, dTTP, dGTP, or one or more analogs thereof. In certain circumstances, 300 to 640 μM dUTP can be substituted for dTTP in the reaction.

The newly synthesized strands form a double-stranded molecule that can be used in the succeeding steps of the reaction. The steps of strand separation, annealing, and elongation can be repeated as often as needed to produce the desired quantity amplification products corresponding to the target MRSA nucleic acid molecule. The limiting factors in the reaction are the amounts of primers, thermostable enzyme, and nucleoside triphosphates present in the reaction. The amplification step and the hybridization step are preferably repeated at least once. For use in detection, the number of amplification and hybridization steps will depend, e.g., on the nature of the sample. If the sample is a complex mixture of nucleic acids, more amplification and hybridization steps may be required to amplify the target sequence sufficient for detection. Generally, the amplification and hybridization steps are repeated at least about 20 times, but may be repeated as many as 40, 60, or even 100 times. In a preferred embodiment of the invention the presence of the FRET within 55, 45 or 35 cycles of amplification and hybridization is indicative of the presence of MRSA in the sample.

The polymerase chain reaction may comprise the steps of adding a thermostable polymerase, nucleotides and primers, whereby a primer is preferably a primer according to the invention, for the target nucleic acid to the sample and thermally cycling the sample between at least a denaturation temperature and an elongation temperature; exciting the sample with light at a wavelength absorbed by the donor fluorescent label and detecting fluorescent emission from the fluorescence energy transfer pair.

The term "thermostable polymerase" refers to a polymerase enzyme that is heat stable, i.e., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T aqualicus, T. lacteus, T rubens, Bacillus stearothermophilus,* and *Methanothermus fervidus.* Nonetheless, polymerases that are not thermostable also can be employed in PCR provided the enzyme is replenished.

In another preferred embodiment of the invention, a method for the detection of a MRSA nucleic acid comprises the steps of amplifying the nucleic acid by polymerase chain reaction in the presence of two nucleic acid probes that hybridize to adjacent regions of the nucleic acid, one of said probes being labeled with an acceptor fluorescent label and the other probe labeled with donor fluorescent label of a fluorescence energy transfer pair such that upon hybridization of the two probes with the target nucleic acid, the donor and acceptor fluorescent labels are within 25 nucleotides of one another, said polymerase chain reaction comprising the steps of adding a thermostable polymerase, nucleotides and primers, whereby the primers are as defined above, for the target nucleic acid to the sample and thermally cycling the sample between at least a denaturation temperature and an elongation temperature; exciting the sample with light at a wavelength absorbed by the donor label and monitoring temperature dependent fluorescence from the fluorescence energy transfer pair.

In another preferred alternative embodiment of the invention, a method for the detection of a MRSA nucleic acid comprises the steps of amplifying the nucleic acid by polymerase chain reaction in the presence of one nucleic acid probe being labeled with an acceptor fluorescent label, e.g. a quencher, and a donor fluorescent label of a fluorescence energy transfer pair, hybridizing the probe with the target nucleic acid, said polymerase chain reaction comprising the steps of adding a polymerase optionally having 5' to 3' exonuclease activity, nucleotides, and primers, whereby the primers are as defined above, for the target nucleic acid to the sample and thermally cycling the sample between at least a denaturation temperature and an elongation temperature; exciting the sample with light at a wavelength absorbed by the donor label and monitoring the fluorescence from the fluorescence energy transfer pair of labels.

For the above-described methods, the nucleic acids can be present in double-stranded or single-stranded form. If the nucleic acid template is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50 %, 60 %, 70 %, 80 %, 90 % or 95 % denatured). The heating conditions necessary for denaturing template nucleic acid will depend, e.g., on the buffer salt concentration and the length and nucleotide composition of the nucleic acids being denatured, but typically range from about 90 °C to about 105 °C for a time depending on features of the reaction such as temperature and the nucleic acid length. Denaturation is typically performed for about 0 sec to 4 min.

In one embodiment of the invention the detecting step is performed after each step of amplification and hybridization and/or in real-time. A detailed description of real-time and on-line monitoring of PCR can be found at http:/

Ibiochem.roche.com/lightcycler. The following patent applications describe real-time PCR as used in the LightCycler technology: WO 97/46707, WO 97/46714 and WO 97/46712. The LightCycler instrument is a rapid thermal cycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the LightCycler thermal chamber. Addition of selected fluorescent dyes to the reaction components allows the PCR to be monitored in real time and on-line. Furthermore, the cuvettes serve as an optical element for signal collection (similar to glass fiber optics), concentrating the signal at the tip of the cuvette. The effect is efficient illumination and fluorescent monitoring of microvolume samples. Exemplary PCR protocols are described in Example 3.

The LightCycler® carousel that houses the cuvettes can be removed from the instrument. Therefore, samples can be loaded outside of the instrument (in a PCR Clean Room, for example). In addition, this feature allows for the sample carousel to be easily cleaned and sterilized. The fluorometer, as part of the LightCycler® apparatus, houses the light source. The emitted light is filtered and focused by an epi-illumination lens onto the top of the cuvette. Fluorescent light emitted from the sample is then focused by the same lens, passed through a dichroic mirror, filtered appropriately, and focused onto data-collecting photohybrids. The optical unit currently available in the LightCycler® instrument (Roche Diagnostics GmBH, Catalog No. 03531414001) includes six band-pass filters (530 nm, Hex excitation, 610 nm, 640 nm, 670 nm, and 705 nm), providing four-color detection and several fluorescence acquisition options. Data collection options include once per cycling step monitoring, fully continuous single-sample acquisition for melting curve analysis, continuous sampling (in which sampling frequency is dependent on sample number) and/or stepwise measurement of all samples after defined temperature interval.

The LightCycler® can be operated using a PC workstation and can utilize a Windows operating system, e.g. Windows XP, Windows NT or Windows 2000. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

In still another embodiment of the invention the method further comprises determining the melting temperature between one of the two probes and the amplification product of step (a), wherein the melting temperature confirms the presence or the absence of MRSA. Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition and its length. Thus, DNA molecules rich in G and C nucleotides and/or longer in nucleotides have a higher Tm than those having an abundance of A and T nucleotides and/or shorter in nucleotides. By detecting the temperature at which signal is lost, the melting temperature of probes can be determined. Similarly, by detecting the temperature at which signal is generated, the annealing temperature of probes can be determined. The melting temperature(s) of the MRSA probes from the respective amplification product can confirm the presence of MRSA in the sample.

In still another embodiment of the invention the method further comprises preventing amplification of a contaminant nucleic acid, particularly comprising performing the amplification step (a) in the presence of uracil, especially comprising treating the sample with uracil-DNA glycosylase prior to a first amplifying step. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing methods of the invention. Containment practices and procedures include, but are not limited to, separate work areas for different steps of a method, containment hoods, barrier filter pipette tips and dedicated air displacement pipettes. Consistent containment practices and procedures by personnel are desirable for accuracy in a diagnostic laboratory handling clinical samples.

Before the nucleic acids of the sample may be analyzed in one of the above-mentioned assays, they might have to be isolated or purified from (biological) samples containing complex mixtures of different components. Often, for the first steps, processes are used which allow the enrichment of the nucleic acids.

In a preferred embodiment of the methods of the invention the sample may be used as crude lysate, which may be prepared as follows:

The sample, e.g. a swab, may be transferred to a suitable buffer and may optionally be treated in order to inactivate bacteria. To release the contents of cells, they may be treated with enzymes or with chemicals to dissolve, degrade or denature the cellular walls. This process is commonly referred to as lysis or mechanical lysis. The resulting solution containing such lysed material is referred to as lysate. The lysate may be purified e.g. by spinning the lysate, discarding the pellet (debris). The supernatant may be used directly as sample in an amplification reaction.

Particularly, the S.E.T.S. kit (Roche Diagnostics Corporation, catalog no. 03753158001) may be use in order to prepare a crude lysate. The method can be exemplarily be described as follows: The sample, e.g. a swab, is broken of the handle and the swab tip is inserted into inner S.E.T.S. tubes (e.g. 0.5 ml tube containing a hole in the bottom and fitting into outer S.E.T.S. tubes, when closed). Tubes are places in outer S.E.T.S. tubes containing silica beads and neutralization buffer (NB). NB neutralizes the transportation medium derived from the swabbing device. Adhering material from the swab is removed from the swab tip and transferred into the outer S.E.T.S. tube by gravitation force (centrifugation). The S.E.T.S. tube is closed and heated to 95°C for heat inactivation of bacteria. Cells are disrupted by mechanical force in a shaking device e.g. the MagNALyser® Instrument. Debris is collected in the bottom of the tube by a short spin and the supernatant is used directly as sample in an amplification reaction. The method is described into more detail in Uhl et al., 2005, J Clin Microbiol. 8:4046-51.

A problem encountered during the lysis is that other enzymes degrading the component of interest, e.g. desoxyribonucleases or ribonucleases degrading nucleic acids, come into contact with the component of interest during lysis. These degrading enzymes may also be present outside the cells or may have been spatially separated in different cellular compartments before the lysis and come now into contact with the component of interest. Other components released during this process may be e.g. endotoxins belonging to the family of lipopolysaccharides which are toxic to cells and can cause problems for products intended to be used in human or animal therapy.

There are a variety of means to tackle this problem mentioned-above. It is common to use chaotropic agents as e.g. guanidinium thiocyanate or anionic, cationic, zwitterionic or nonionic detergents when nucleic acids are intended to be set free. It is also an advantage to use proteases which rapidly degrade these enzymes or unwanted proteins. However, this may produce another problem as the said substances or enzymes can interfere with reagents or components in subsequent steps.

Enzymes which can be advantageously used in such lysis or sample preparation processes mentioned-above are enzymes which cleave the amide linkages in protein substrates and which are classified as proteases, or (interchangeably) peptidases (see Walsh, 1979, Enzymatic Reaction Mechanisms. W. H. Freeman and Company, San Francisco, Chapter 3). Proteases which have been used in the prior art are e.g. alkaline proteases (WO 98/04730) or acid proteases (US 5,386,024). The protease which is widely used in the prior art for sample preparation for the isolation of nucleic acids is proteinase K from *Tritirachium album* (see e.g. Sambrook J. et al., Molecular Cloning: A Laboratory Manual, Cold Spring Harbor Laboratory Press, Cold Spring Harbor, New York, 1989) which is active around neutral pH and belongs to a family of proteases known to the person skilled in the art as subtilisins.

In the next steps of the sample preparation which follow on the lysis step, the component of interest can be further enriched. If the non-proteinaceous components of interest are e.g. nucleic acids, they are normally extracted from the complex lysis mixtures before they are used in a probe-based assay.

There are several methods for the extraction of nucleic acids:

- sequence-dependent or biospecific methods as e.g.:
    - affinity chromatography
    - hybridisation to immobilised probes
- sequence-independent or physico-chemical methods as e.g.:
    - liquid-liquid extraction with e.g. phenol-chloroform
    - precipitation with e.g. pure ethanol
    - extraction with filter paper
    - extraction with micelle-forming agents as cetyl-trimethyl-ammonium-bromide
    - binding to immobilised, intercalating dyes, e.g. acridine derivatives
    - adsorption to silica gel or diatomic earths
    - adsorption to magnetic glass particles (MGP) or organo silane particles under chaotropic conditions

Particularly interesting for extraction purposes is the adsorption of nucleic acids to a glass surface although other surfaces are possible. Many procedures for isolating nucleic acids from their natural environment have been proposed in recent years by the use of their binding behavior to glass surfaces. If unmodified nucleic acids are the target, a direct binding of the nucleic acids to a material with a silica surface is preferred because among other reasons the nucleic acids do not have to be modified and even native nucleic acids can be bound. These processes are described in detail by various documents. In Vogelstein et al., 1979, Proc. Natl. Acad. U.S.A. 76: 615-619, for instance, a procedure for binding nucleic acids from agarose gels in the presence of sodium iodide to ground flint glass is proposed. The purification of plasmid DNA from bacteria on glass dust in the presence of sodium perchlorate is described in Marko et al., 1982, Anal. Biochem. 121: 382-387. In DE-A 37 34 442, the isolation of single-stranded M13 phage DNA on glass fiber filters by precipitating phage particles using acetic acid and lysis of the phage particles with perchlorate is described. The nucleic acids bound to the glass fiber filters are washed and then eluted with a methanol-containing Tris/EDTA buffer. A similar procedure for purifying DNA from lambda phages is described in Jakobi R. et al., Anal. Biochem. 175 (1988) 196-201. The procedure entails the selective binding of nucleic acids to glass surfaces in chaotropic salt solutions and separating the nucleic acids from contaminants such as agarose, proteins or cell residue. To separate the glass particles from the contaminants, the particles may be either centrifuged or fluids are drawn through glass fiber filters. This is a limiting step, however, that prevents the procedure from being used to process large quantities of samples. The use of magnetic particles to immobilize nucleic acids after precipitation by adding salt and ethanol is more advantageous and described e.g. in Alderton et al., 1992, Anal. Biochem. 201: 166-169. In this procedure, the nucleic acids are agglutinated along with the magnetic particles. The agglutinate is separated from the original solvent by applying a magnetic field and performing a wash step. After one wash step, the nucleic acids are dissolved in a Tris buffer. This procedure has a disadvantage, however, in that the precipitation is not selective for nucleic acids. Rather, a variety of solid and dissolved substances are agglutinated as well. As a result, this procedure can not be used to remove significant quantities of any inhibitors of specific enzymatic reactions that may be present. Magnetic, porous glass is also available on the market that contains magnetic particles in a porous, particular glass matrix and is covered with a layer containing streptavidin. This product can be used to isolate biological materials, e.g., proteins or nucleic acids, if they are modified in a complex preparation step so that they bind covalently to biotin. Magnetizable particular adsorbents proved to be very efficient and suitable for automatic sample preparation. Ferrimagnetic and ferromagnetic as well as superparamagnetic pigments are used for this purpose. The most preferred MGPs and methods using magnetic glass particles are those described in WO 01/37291.

After the purification or isolation of the nucleic acids including the target nucleic acid from their natural surroundings, the target nucleic acid, the MRSA-specific nucleic acid, may be detected.

Within each thermocycler run, control samples can be cycled as well. Accordingly, in a further embodiment of the invention, the method of the invention includes a control sample, particularly wherein the control sample comprises MRSA nucleic acid molecule. Control nucleic acid template can be amplified from a positive control (reagent control) sample using, for example, control primers and control probes. Positive control samples can also be used to amplify, for example, a plasmid construct containing MRSA nucleic acid molecules. Such a plasmid control can be amplified internally (e.g., within each biological sample) (internal control) or in separate samples run side-by-side with the patients' samples. Each thermocycler run also should include a negative control that, for example, lacks MRSA nucleic acid. Such controls are indicators of the success or failure of the amplification, hybridization, and/or FRET reaction. Therefore, control reactions can readily determine, for example, the ability of primers to anneal with sequence-specificity and to initiate elongation, as well as the ability of probes to hybridize with sequence-specificity and for FRET to occur.

A preferred control concept comprises a positive control (detecting a known MRSA DNA), a reagent control (detecting a plasmid comprising the MRSA target sequence) and an internal control (detecting a plasmid comprising the MRSA target sequence, wherein the probe binding site, e.g. that for the MRSA sensor probe, has been substituted by another sequence which does not bind the MRSA sensor probe but which is recognised by the internal control sensor probe; alternatively, the binding sites for both probes may be exchanged). The internal control may be part of each of the reactions, i.e. positive control, reagent control negative control and each sample.

An example of a suitable probe for internal control (IC) is:

MRSA IC 4: 5'- CCA GCA GAA TGC CAA CCA -3' (SEQ ID NO: 91), e.g. 5' labelled with LC-Red 670 or
MRSA direct IC: 5'- CCA GCA GAA TGC CAG CCA AT -3' (SEQ ID NO: 98), e.g. 5' labelled with LC-Red 670.

The sample to be analyzed may be any sample. However, the sample will in general be a biological sample, preferably a sample from a human subject. Representative biological samples that can be used in practicing the methods of the invention include swabs or body fluids such as a sample selected from the group consisting of a swab of an infected wound, a skin swab, a nasal swab, a throat swab, a groin swab, a axilla swab, a swab from site of an invasive device, a swab from site of possible infection, a blood sample, a urine sample and a perineum swab. Biological sample collection and storage methods are known to those of skill in the art. Inadequate specimen collection, transportation delays, inappropriate transportation conditions, or use of certain collection swabs (e.g., calcium alginate or aluminum shaft) are all conditions that can affect the success and/or accuracy of the test result. Biological samples can be processed (e.g., by standard nucleic acid extraction methods and/or using commercial kits) to release MRSA nucleic acid, the biological sample is contacted directly with the PCR reaction components and the appropriate oligonucleotides.

A further subject of the disclosure relates to a primer, a set of primers, a probe and/or a pair of probes as defined in any of the above embodiment of the invention.

Still a further subject of the present invention relates to a kit comprising:

(a) a set of primers as defined in any of type RE7; appended claims 1-5,

(b) a pair of probes or a probe as defined in any of appended claims 6 or 8 or a probe as defined in appended claim 10, and

(c) a donor fluorescent moiety and a corresponding fluorescent moiety labeling the probe(s),

wherein the kit is capable of detecting each of Staphylococcal Chromosomal Cassettes (SCC*mec*) types I to V of MRSA.

The set of primers, probe and/or pair of probes may be as described for the above (preferred) embodiments or suitable for any of the methods of the invention described above. Additionally, the kit may comprise a package label or package insert having instructions thereon for using the set of MRSA primers and the pair of MRSA probes to detect the presence or absence of in a sample. Further optional components of the kit of the invention are at least one suitable enzyme, particularly uracil-DNA-glycosylase and/or a DNA polymerase, and/or a suitable buffer. The kit may also contain a template-dependent polymerase having 3' to 5' exonucleolytic activity, preferably the Taq Polymerase, nucleotides and oligonucleotides. In another embodiment of the invention, a kit is provided comprising a template dependent DNA polymerase, nucleotides and an oligonucleotide or a pair of primers according to the invention.

Such kits known in the art further comprise plastics ware which can be used during the amplification procedure as e.g. microtitre plates in the 96 or 384 well format or just ordinary reaction tubes manufactured e.g. by Eppendorf, Hamburg, Germany and all other reagents for carrying out the method according to the invention.

In another embodiment of the invention, the kit contains further reagents for isolating the nucleic acid. Therefore, the kit can additionally contain a material with an affinity to nucleic acids, preferably the material with an affinity to nucleic acids comprises a material with a silica surface. Preferably, the material with a silica surface is a glass. Most preferably, the material with an affinity to nucleic acids is a composition comprising magnetic glass particles as described in WO 96/41811 or WO 01/37291. The kit can further or additionally comprise a lysis buffer containing e.g. chaotropic agents, detergents or alcohols or mixtures thereof which allows the lysis of cells and separately a protease, e.g. proteinase K, for the digestions of unwanted proteins. These components of the kit according to the invention may be provided separately in tubes or storage containers. Depending on the nature of the components, these may be even provided in a single tube or storage container. The kit may further or additionally comprise a washing solution which is suitable for the washing step of the magnetic glass particles when DNA or RNA is bound thereto. This washing solution may contain ethanol and/ or chaotropic agents in a buffered solution or solutions with an acidic pH without ethanol and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the DNA or RNA bound to the magnetic glass particles. Further, additional reagents or buffered solutions may be present which can be used for the purification process of a nucleic acid, i.e. DNA or RNA.

In a preferred embodiment of the invention the kit contains inner, outer S.E.T.S. tubes and/or the neutralization buffer as sample prep reagents. Preferably, the method of the invention is carried out without purification of the samples by preparing crude lysates, e.g. as detailed above. Accordingly, the kit may be adapted to this method in that means for further isolation or purification of nucleic acids are not included into the kit.

A preferred embodiment of the present invention is to use the method or the kit of the present invention in automatable methods as e.g. described in WO 99/16781. Automatable method means that the steps of the method are suitable to be carried out with an apparatus or machine capable of operating with little or no external control or influence by a human being. Automatized method means that the steps of the automatable method are carried out with an apparatus or machine capable of operating with little or no external control or influence by a human being. Only the preparation steps for the method may have to be done by hand, e.g. the storage containers have to filled up and put into place, the choice of the samples has to be done by a human being and further steps known to the expert in the field, e.g. the operation of the controlling computer. The apparatus or machine may e.g. add automatically liquids, mix the samples or carry out incubation steps at specific temperatures. Typically, such a machine or apparatus is a robot controlled by a computer which carries out a program in which the single steps and commands are specified. Preferred automatized methods are those which are carried out in a high-throughput format which means that the methods and the used machine or apparatus are optimized for a high-throughput of samples in a short time. In another embodiment of the invention the methods or the kits according to the present invention are used in semi-automatized process which means that some reaction steps may have to be done manually. In a preferred embodiment of the invention, a suspension containing MGPs according to the present invention is taken from a storage container and partial volumes are added to different reaction vessels. Reaction vessels may be reaction tubes made from plastics eventually in microtitre plate format contain 96 or 384 or more wells where a reaction can be carried out. However, these vessels may be made from other material e.g. from steel.

In preferred embodiments of the invention the kit according to the invention is used for research, bioanalytics or diagnostics. In preferred embodiments according to the invention the kit according to the invention or the method according to the invention is used in a high-throughput format, i.e. in an automatized method which allows the analysis of a high number of different samples in a very short time.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims. Additionally, it is understood that the present invention is not limited by the configuration of one or more commercially available instruments, particularly those described herein.

**FIGURES**

Fig. 1 shows amplicon sequences for MRSA, particularly RE2, RE3 and RE7 (each including the beginning of orfX (**CC**)) as well as orfX.

---------- primer
........... first probe (anchor probe)
-- -- -- second probe (sensor probe)
indicates a mismatch

Fig. 2 shows the sequence of RE2 alignment from 93 different MRSA strains. Point mutations including the number of mutations and the kind of alterations are given. A 102 bp duplication present in SCC*mec* type I is identified by. The transition from SCC*mec* to the SA portion of the sequence is identified by **CC**.

Fig. 3 shows amplification curves (Fig. 3A) and melting curves (Fig. 3B) for RE2 detection. In analytical experiments a dynamic range of 5 log steps and a sensitivity in the range of 10 genomic copies proved fulfilment of requirements for diagnostic use. This experiments proves functionality of the assay.

Fig. 4 shows amplification curves (Fig. 4A) and melting curves (Fig. 4B) for RE3 detection. In analytical experiments a dynamic range of 5 log steps and a sensitivity in the range of 10 genomic copies proved fulfilment of requirements for diagnostic use. This experiments proves functionality of the assay.

Fig. 5 shows amplification curves (Fig. 5A) and melting curves (Fig. 5B) for RE7 detection. In analytical experiments a dynamic range of 5 log steps and a sensitivity in the range of 10 genomic copies proved fulfilment of requirements for diagnostic use. This experiments proves functionality of the assay.

**EXAMPLES**

**EXAMPLE 1: DIAGNOSTIC IN VITRO TEST FOR MRSA**

The LightCycler® MRSA direct test is a qualitative *in vitro* diagnostic test for the direct detection of methicillin resistant *Staphylococcus aureus* (MRSA) to aid in the prevention and control of MRSA colonization in e.g. healthcare settings. The test performed on the LightCycler® 2.0 Instrument with specimens from patients suspected for colonization, utilized swab extraction and mechanical lysis for specimen preparation followed by polymerase chain reaction (PCR) for the amplification of MRSA DNA and fluorogenic target specific hybridization probes for the detection of the amplified DNA.

Instrument Requirements: For this assay the LightCycler® 2.0 is specified.

Reagent Requirements: The assay consisted of the sample prep reagents and amplification reagents. Sample preparation was carried out by extraction of bacteria from swabs and a subsequent lysis step. Lysis can be done either enzymatically or mechanically. For SA mechanical lysis showed good results in the past. In addition mechanical lysis can be done irrespective of the bacteria or bacterial phenotype present in the sample. Therefore, mechanical lysis was the favored method. For further processing of the lysate two options are possible.

1. Sample lysis with subsequent DNA extraction
2. Sample lysis and use of crude extracts

Since time to result was important and just lysis is fast, preferably no DNA purification was done only a crude lysate was generated and directly used for PCR.

The assay used amplification by PCR and detection using FRET probes. Controls for amplification and internal controls were included, in order to control amplification and hybridization. Detection was either by amplification curve or melting curve analysis. Software Requirements: The software SW 4.05 was used for data analysis of the MRSA direct test. For data analysis two possible algorithms may be used.

When amplification curves were used (qualitative detection module) data output was achieved by the LightCycler SW without further manual steps. Programming, the control concept and the display of the outcome was steered via a macro. For this solution data output relied on the SW intrinsic algorithm. The data outcome was not controlled via an expert analysis.

Melting curve analysis required a manual analysis step to be performed by the customer. The advantage was a better specificity.

Workflow description: The workflow for the MRSA direct test encompassed:

1. Swab extraction using the Neutralization buffer, inner and outer S.E.T.S. tubes.
2. Lysis of the bacteria including addition of the internal control (IC) via the neutralization buffer (Heat and mechanical disrupt).
3. Transfer of the crude lysate (specimen extract) to the Reaction Mix (RM). The RM consisted of the primer and probes, the FastStart enzyme (Taq polymerase), UNG (uracil-N-glycosilase) and buffer.
4. Amplification and detection using specific primer and probes (PCR).
5. Data analysis in two steps. First validation of the experiment via the respective control. Second identification of the presence or absence of the target analyte.

**EXAMPLE 2: Design of Primer and Probes**

The primer and probes were designed so that the reverse primer and the pair of probes (detection probes) are located in the *S. aureus* portion of the target region. Therefore, this element remained the same for all RE types. RE types describe the sequence variations of the right extremity junction of the SCC *mec* transposon. The RE2 amplicon representing the majority of all RE types has been evaluated by sequencing 93 unrelated specimens (Fig. 2). For this predominant RE type (representing SCC*mec* type I, II, III (some) and some IV) primers and probes have been designed (see table 1). This setup represents the core assay. Further primers in order to cover SCC types I to V were added when appropriate. (see table 1). For primer and probe design LightCycler® Probe Design Software 2.0 was used. Variants of these primers and probes were designed in order to optimize signal output (see table 1).

**Table 1: Primers and Probe design for SCCmec RE2, RE3 and RE7 detection**

| ID | Label | Sequence (without label) | (SEQ ID NO:) |
|---|---|---|---|
| Primer/Probe Primers for detection of RE2 | | | |
| AR *mec* 1 fwd | none | 5'- GCA ATT CAC ATA AAC CTC ATA TGT TC -3' | (1) |
| AR *mec* 2 fwd | none | 5'- ACC TCA TAT GTT CTG ATA CAT TCA -3' | (2) |
| AR *mec* 3 fwd | none | 5'- GCA ATT CAC ATA AAC CTC ATA T -3' | (3) |
| AR *mec* 4 fwd | none | 5'- CAT AAC AGC AAT TCA CAT AAA CCT C -3' | (4) |
| AR *mec* 5 fwd | none | 5'- TAA CAG CAA TTC ACA TAA ACC T -3' | (5) |
| AR *mec* 6 fwd | none | 5'- CGC TAT TAT TTA CTT GAA ATG AAA GAC -3' | (6) |
| AR *mec* 7 fwd | none | 5'- CTT GAA ATG AAA GAC TGC GGA -3' | (7) |
| AR *mec* 8 fwd | none | 5'- TTG CTT CAC TAT AAG TAT TCA GTA TAA AGA -3' | (8) |
| AR *mec* 9 fwd | none | 5'- ATT TAC TTG AAA TGA AAG ACT GCG -3' | (9) |
| AR *mec* 10 fwd | none | 5'- AAA GAA TAT TTC GCT ATT ATT TAC TTG AA -3' | (10) |
| AR *mec* 11 fwd | none | 5'- TCA GTA TAA AGA ATA TTT CGC TAT TAT TT -3' | (11) |
| AR *mec* 12 fwd | none | 5'- TGA AAT GAA AGA CTG CGG AG -3' | (12) |
| JU1 fwd | none | 5'- AAC CTC ATA TGT TCT GAT ACA TTC AAA -3' | (13) |
| JU2 fwd | none | 5'- TAT GTC AAA AAT CAT GAA CCT CAT TAC T -3' | (14) |
| JU3 fwd | none | 5'- CAT AAC AGC AAT TCA CAT AAA CCT C -3' | (15) |
| JU4 fwd | none | 5'- GAC TGC GGA GGC TAA CT -3' | (16) |
| JU5 fwd | none | 5'- ATC CCT TTA TGA AGC GGC -3' | (17) |
| MRSA direct RE2 fwd | none | 5'- TGA AAT GAA AGA CTG CGG AG -3' | (92) |

(continued)

| | | | |
|---|---|---|---|
| Primers for detection of RE3 | | | |
| AR *mecA* 3/1 | none | 5'- GCA AGG TAT AAT CCA ATA TTT CAT ATA TGT -3' | (18) |
| AR *mecA* 3/2 | none | 5'- AGT TCC ATA ATC AAT ATA ATT TGT ACA GT -3' | (19) |
| AR *mecA* 3/3 | none | 5'- ACA TCG TAT GAT ATT GCA AGG TA -3' | (20) |
| AR *mecA* 3/4 | none | 5'- CTT TCA TTC TTT CTT GAT TCC ATT AG -3' | (21) |
| AR *mecA* 3/5 | none | 5'- CAC TCT ATA AAC ATC GTA TGA TAT TGC -3' | (22) |
| AR *mecA* 3/6 | none | 5'- TTC TTA ATT TAA TTG TAG TTC CAT AAT CAA -3' | (23) |
| AR *mecA* 3/7 | none | 5'- AAT TAT ACA CAA CCT AAT TTT TAG TTT TAT -3' | (24) |
| AR *mecA* 3/8 | none | 5'- AAT TTT TAG TTT TAT TTA TGA TAC GCT TC -3' | (25) |
| AR *mecA* 3/9 | none | 5'- ACA CAA CCT AAT TTT TAG TTT TAT TTA TGA -3' | (26) |
| AR *mecA* 3/10 | none | 5'- TTT ATT AAA CAC TCT ATA AAC ATC GTA TGA -3' | (27) |
| AR *mecA* 3/13 | none | 5'- CCA CAT CTC ATT AAA TTT TTA AAT TAT ACA C -3' | (28) |
| MRSA direct RE3 fwd | none | 5'- CCA CAT CTC ATT AAA TTT TTA AAT TAT ACA C -3' | (93) |
| Primers for detection of RE7 | | | |
| AR *mec* 5/1 | none | 5'- ATA TTA TAC ACA ATC CGT TTT TTA GTT TTA -3' | (29) |
| AR *mec* 5/2 | none | 5'- ACA CAA TCC GTT TTT TAG TTT TAT TTA TG -3' | (30) |
| AR *mec* 5/3 | none | 5'- TTC TAA TTT ATT TAA CAT AAA ATC AAT CCT -3' | (31) |
| AR *mec* 5/16 | none | 5'- CAA TCC TTT TTA TAT TTA AAA TAT ATT ATA CAC -3' | (32) |
| MRSA direct RE7 fwd | none | 5'- CAA TCC TTT TTA TAT TTA AAA TAT ATT ATA CAC -3' | (94) |
| Primers specific for MRSA/SSRA | | | |
| AR *mec* 1 rev | none | 5'- AGG AAA GAT GCT ATC TTC CGA -3' | (33) |
| *AR mec 2 rev* | none | 5'- GAA AGA TGC TAT CTT CCG AAG -3' | (34) |
| AR *mec* 3 rev | none | 5'- GAT GCT ATC TTC CGA AGG -3' | (35) |
| AR *mec* 4 rev | none | 5'- GTC ATT ACA TTA GAA ATA CAA GGA AAG AT -3' | (36) |
| AR *mec* 5 rev | none | 5'- GCC AAC GAA TAC TAG CC -3' | (37) |
| AR *mec* 6-2 rev | none | 5'- ACG AAT ACT AGC CAA AAT TAA ACC -3' | (38) |
| *AR mec 7 rev* | none | 5'- CAC AAT CCA CAG TCA TTA CAT TAG A -3' | (39) |
| JU1 rev | none | 5'- CAA GGA AAG ATG CTA TCT TCC G -3' | (40) |
| JU2 rev | none | 5'- AGT CAT TAC ATT AGA AAT ACA AGG AAA GA -3' | (41) |
| JU3 rev | none | 5'- AGGAAAGATGCTATCTTCCGA -3' | (42) |
| JU4 rev | none | 5'- AGG AAA GAT GCT ATC TTC CGA -3' | (43) |
| JU5 rev | none | 5'- ACA ATC CAC AGT CAT TAC ATT AGA A -3' | (44) |
| MRSA direct rev | none | 5'- CAA GGA AAG ATG CTA TCT TCC G -3' | (95) |
| Probes specific for orfX | | | |
| AR *mec* Fluo 1 | Fluos | 5'- AAG TCG CTT TGC CTT TGG GTC A -3' | (45) |
| AR *mec* Fluo 2 | Fluos | 5'- TAC AAA GTC GCT TTG CCT TTG GGT CA -3' | (46) |
| AR *mec* Fluo 3 | Fluos | 5'- GGC CGT TTG ATC CGC CAA T -3' | (47) |
| AR *mec* Fluo 4 | Fluos | 5'- AAG TCG CTT TGC CCT TGG GTA -3' | (48) |
| *AR mec* Fluo 4-2 | Fluos | 5'- AAG TCG CTT TGC CCT TGG GT -3' | (49) |
| AR *mec* Fluo 4-3 | Fluos | 5'- AAG TCG CTT TGC CCT TGG GTC A -3' | (50) |
| AR *mec* Fluo 4-GV | Fluos | 5'- AAG TCG CTT TGC CCT TGG G -3' | (51) |

(continued)

| Probes specific for orfX | | | |
|---|---|---|---|
| AR *mec* Fluo 4k | Fluos | 5'-CAA GAA TTG AAC CAA CGC AT -3' | (52) |
| AR *mec* Fluo 5 | Fluos | 5'- CAA TGA CGA ATA CAT AGT CGC TTT GCC CTT -3' | (53) |
| AR *mec* Fluo 6 | Fluos | 5'- CGT TTG ATC CGC CAA TGA CGA -3' | (54) |
| AR *mec* Fluo 7 | Fluos | 5'- GCC AAT CCT TCG GAA GAT AGC A -3' | (55) |
| AR *mec* Fluo UR | Fluos | 5'- ATT AAC ACA ACC CGC ATC -3' | (56) |
| JU1 probe 1 | Fluos | 5'- GTC GCT TTG CCC TTG GGT C -3' | (57) |
| JU2 probe 1 | Fluos | 5'- TCG CTT TGC CCT TGG GTC AT -3' | (58) |
| JU3 probe 1 | Fluos | 5'- GGC CGT TTG ATC CGC CAA T -3' | (59) |
| JU4 probe1 | Fluos | 5'- GTC CTT GTG CAG GCC GTT TGA T -3' | (60) |
| JU5 probe1 | Fluos | 5'- CTT GGG TCA TGC GTT GGT TCA ATT -3' | (61) |
| MRSA direct Fluos | Fluos | 5'- AAG TCG CTT TGC CCT TGG G -3' | (96) |
| AR *mec* 640 3 | LC Red | 5'- CGA ATA CAA AGT CGC TTT GCC CTT GGG -3' | (62) 640 |
| AR *mec* 640 4 | LC Red 640 | 5'- ATG CGT TGG TTC AAT TCT TG -3' | (63) 640 |
| AR *mec* 610 4-3 | LC Red 610 | 5'- GCG TTG GTT CAA TTC TTG GG -3' | (64) |
| AR *mec* 640 4k | LC Red 640 | 5'- ACC CAA GGG CAA AGC GAC TT -3' | (65) |
| AR *mec* 640 5 | LC Red 640 | 5' - GGT AA T GCG TTG GTT CAA TTC TTG -3' | (66) |
| AR *mec* 640 6 | LC Red 640 | 5'- ACA AAG TCG CTA TGC CCT TGG GTC A -3' | (67) |
| AR *mec* 640 7 | LC Red 640 | 5'- CTT TCC TTG TAT TTC T AA TGT AA T GAC TG -3' | (68) |
| AR *mec* 640 UR | LC Red 640 | 5'- TTG ATG TGG GAA TGT CAT TTT GCT GAA -3' | (69) |
| JU1 probe 2 | LC Red 640 | 5'- GCG TTG GTT CAA TTC TTG GGC CAA T -3' | (70) |
| JU2 probe 2 | LC Red 640 | 5'- GTT GGT TCA ATT CTT GGG CCA ATC CTT CG -3' | (71) |
| JU3 probe 2 | LC Red 640 | 5'- CGA ATA CAA AGT CGC TTT GCC CTT GG -3' | (72) |
| JU4 probe2 | LC Red 640 | 5'- GCC AAT GAC GAA TAC AAA GTC GCT TTG CC -3' | (73) |
| JU5 probe2 | LC Red 640 | 5'- TGG GCC AAT CCT TCG GAA GAT AGC A -3' | (74) |
| AR *mec* 610 4-MM2 | LC Red 640 | 5'- ATG CGT TGG TTC GAT TCT TG -3' | (75) |
| AR *mec* 610 4-MM2-GV | LC Red 610 | 5'- CAT GCG TTG GTT CGA TTC TTG -3' | (76) |
| MRSA direct Red610 | LC Red 610 | 5'- CAT GCG TTG GTT CGA TTC TTG -3' | (97) |

**EXAMPLE 3: Analysis of Particular Primers and Probes**

For RE2, RE3 and RE7 single tests using the same probes and the same reverse primer but different forward primers were established (see table 1). These single tests were analyzed for the dynamic range from about $10^6$ genomic copies/PCR to 10 copies/PCR in tenfold dilutions (Figures 3 to 5). For data analysis the qualitative detection module and manual Tm calling were employed.

<u>Reagent Concentrations:</u>

forward primers: 0.2 - 0.5 μM
reverse primer: 0.3 - 0.5 μM
probes: 0.1-0.4 μM
$MgCl_2$: 3.0 - 4.5 mM (Final concentration needs to be adapted depending on the $MgCl_2$ concentration present in the resp. Reaction Mix)
LightCycler® Uracil DNA Glycosylase: 1 unit/reaction
LightCyler® 480 Probes Master resp. LightCycler® FastStart DNA Master HybProbe resp.
LightCycler® Fast Start DNA Master $^{PLUS}$ HybProbe

PCR protocol:

| | temperature | hold | ramp rate | acquisition |
|---|---|---|---|---|
| denaturation (1 cycle): | 95°C | 10 min | 20 | none |
| amplification (45 cycles): | 95°C | 10 sec | 20 | none |
| | 52-55°C | 10 sec | 20 | single |
| | 72°C | 12-22 sec | 20 | none |
| melting curve (1 cycle) : | 95°C | 0 sec | 20 | none |
| | 54°C | 20 sec | 20 | none |
| | 45°C | 20 sec | 0.2 | none |
| | 80°C | 0 sec | 0.1 | cont. |
| cooling (1 cycle) : | 40°C | 30 sec | 20 | none |

**Table 2: Primers and Probe used in figures 3-5 for SCCmec RE2, RE3 and RE7 detection**

| Name | Function | Label | Sequence (SEQ ID NO:) |
|---|---|---|---|
| AR *mec* 12 fwd | RE2 detection | | 5'- TGA AAT GAA AGA CTG CGG AG -3' (12) |
| AR *mecA 3*/*13* | RE3 detection | | 5'- CCA CAT CTC ATT AAA TTT TTA AAT TAT ACA C -3' (28) |
| AR *mec* 5/16 | RE7 detection | | 5'- CAA TCC TTT TTA TAT TTA AAA TAT ATT ATA CAC -3' (32) |
| JU1 rev: | Specificity for SA | | 5'- CAA GGA AAG ATG CTA TCT TCC G -3' (40) |
| AR *mec* Fluo 4-GV: | Specificity for MRSA | Flous | 5'- AAG TCG CTT TGC CCT TGG G -3' (51) |
| AR *mec* 610 4-MM2-GV: | Specificity for MRSA | LC-Red 610 | 5'- CAT GCG TTG GTT CGA TTC TTG -3' (76) |
| RSA IC 4 | Internal Control | LC-Red 670 | 5'- CCA GCA GAA TGC CAA CCA -3' (91) |

Reagent Concentrations used in figures 3 - 5:

*AR mec* 12 fwd: 0.5 μM
AR *mecA* 3/13: 0.3 μM
AR *mec* 5/16: 0.3 μM
JU1 rev: 0.5 μM
AR *mec* Fluo 4-GV: 0.2 μM
AR *mec* 610 4-MM2-GV: 0.2 μM
LightCycler® Uracil DNA Glycosylase: 1 unit/reaction
LightCyler® 480 Probes Master (final concentration in the PCR reaction is 3.2 mM $MgCl_2$.)

PCR protocol used in figures 3 - 5:

| | temperature | hold | ramp rate | acquisition |
|---|---|---|---|---|
| denaturation (1 cycle): | 95°C | 10 min | 20 | none |

(continued)

| | temperature | hold | ramp rate | acquisition |
|---|---|---|---|---|
| amplification (45 cycles): | 95°C | 10 sec | 20 | none |
| | 52 °C | 10 sec | 20 | single |
| | 72°C | 18 sec | 20 | none |
| melting curve (1 cycle) : | 95°C | 0 sec | 20 | none |
| | 54°C | 20 sec | 20 | none |
| | 45°C | 20 sec | 0.2 | none |
| | 80°C | 0 sec | 0.1 | cont. |
| cooling (1 cycle): | 40°C | 30 sec | 20 | none |

A further Example was performed as described above with the exception that the following primers and probes were used:

| | |
|---|---|
| AR *mec* 11 fwd: | for RE2 detection |
| AR *mecA* 3/8: | for RE3 detection |
| AR *mec* 5/2: | for RE7 detection |
| AR *mec* 6-2 rev: | Specificity for SA |
| AR *mec* Fluo 4: | Specificity for MRSA |
| AR *mec* 610 4-MM2: | Specificity for MRSA |

In this example curves similar to those of figures 3 to 5 were obtained and Tm values of 57.21 °C, 56.71 °C and 56.89 °C were calculated for RE2, RE3 and RE7, respectively.

**EXAMPLE 4: Analysis of the LC MRSA Assay System of the invention**

A PCR assay for methicillin-resistant Staphylococcus aureus (MRSA) present on nasal swab specimens was developed with primers for amplification and FRET hybridization probes for detection of the amplified target nucleic acid with the LightCycler instrument. The assay was performed as described in the above Examples 1 to 3 unless otherwise noted.

This example describes the performance evaluation of a preferred set of primers and probes (Roche LC MRSA assay system (PCR)).

| Oligo name | Label | Sequence (5' - 3') | SEQ ID NO: |
|---|---|---|---|
| Primer, MRSA direct RE2 fwd | None | TGA AAT GAA AGA CTG CGG AG | 92 |
| Primer, MRSA direct RE3 fwd | None | CCA CAT CTC ATT AAA TTT TTA AAT TAT ACA C | 93 |
| Primer, MRSA direct RE7 fwd | None | CAA TCC TTT TTA TAT TTA AAA TAT ATT ATA CAC | 94 |
| Primer, MRSA direct rev | None | CAA GGA AAG ATG CTA TCT TCC G | 95 |
| Probe, MRSA direct Fluos | 3' Fluorescein | AAG TCG CTT TGC CCT TGG G | 96 |
| Probe, MRSA direct Red 610 | 5' LC Red 610, 3' Phosphat | CAT GCG TTG GTT CGA TTC TTG | 97 |
| Probe, MRSA direct IC Red 670 | 5' LC Red 670, 3' Phosphat | CCA GCA GAA TGC CAG CCA AT | 98 |

Workflow:

Nasal swabs delivered to the laboratory are broken off into a screw capped lysis tube containing 600 μl of neutralization buffer and approximately 50 μl of 0.1 mm glass beads. The tube is capped, heated for 2 minutes in a heating block set at 95 to 100C and processed on a MagNA Lyser for 70 seconds at a speed setting of 5000. The tube is centrifuged at 20,000 x g for 1 minute and 5 μl of the liquid above the swab is used for PCR of MRSA.

PCR:

Three types of MRSA (RE2, RE3 and RE7) are detected with the PCR assay. Melting curve analysis of the 640 nm signal is used to detect MRSA. All three types of MRSA provide the same melting curve melting temperature. Melting curves above baseline with a $T_m$ within +/- 2 °C of the positive control are considered positive. An internal control (IC) template is included in the assay using hybridization probe detection at 710 nm. Detection of the positive control in negative specimens is necessary to demonstrate lack of PCR inhibition.

• Specificity

Coagulase negative staphylococcus may have a portion of the PCR target. To confirm that isolates of these bacteria do not cross-react in the PCR, DNA from lysed cultures of 29 mecA-negative and 75 mecA-positive coagulase-negative staphylococci were tested. None of the coagulase-negative staphylococci gave a positive result.

One hundred clinical isolates of S. aureus which are sensitive to methicillin (MSSA) were tested with PCR to determine if they give a false positive result with PCR. None of the S. aureus isolates gave a positive PCR result.

• Inclusivity

DNA from lysed colonies of MRSA isolates from the US (207) and South Africa (105) were tested by PCR. Four MRSA isolates (1.3%) were not detected by PCR. Five MRSA isolates were detected with reduced sensitivity.

• Exogenous interference

PCR inhibition was tested by adding various amounts of whole blood to a swab and processing according to the workflow. Spiking with 5 or 10 μl of whole blood showed no inhibition of the internal control. Adding 20 μl of blood gave less than 10 % inhibition from replicate tests. Adding 70 μl of whole blood to the swab produced inhibition of PCR of the internal control with most replicate tests.

• Sensitivity

The sensitivity of the PCR for the RE2, RE3 and RE7 MRSA targets was evaluated by testing in replicates of 5 the number of positive PCR results for dilutions of DNA from each MRSA type.

| | MRSA type | | |
|---|---|---|---|
| DNA copies | RE2 | RE3 | RE7 |
| 20 | 5 | 5 | 5 |
| 10 | 5 | 4 | 5 |
| 5 | 3 | 4 | 3 |
| 2.5 | 0 | 1 | 1 |

The sensitivity was ≥ 90% for all three MRSA types when 10 copies of DNA were used for PCR.

• Clinical Sensitivity and Specificity

A clinical trial with 165 nasal swab specimens was performed. The gold standard for comparison was the culture-based detection method using CHROMagar MRSA (BD, Becton, Dickinson and Company, NJ, USA). Three swabs were found to be inhibitory to PCR. The following results were found with the remaining 162 swabs.

| | | CHROMagar MRSA | |
|---|---|---|---|
| | | Pos | Neg |
| MRSA PCR | Pos | 33 | 10* |
| | Neg | 1 | 118 |
| * 5 of 10 were on antibiotic treatment at the time of testing. | | | |

| | All patients | Patients not on antibiotics# |
|---|---|---|
| Sensitivity | 97.1% | 98.0% |
| Specificity | 92.2% | 95.2% |
| PPV | 76.7% | 90.9% |
| NPV | 99.2% | 99.0% |
| # Excluding patients that were not on antibiotics which may give a false negative result to the culture-based CHROMagar MRSA | | |

• Swab types

Three swab types were evaluated for use with MRSA PCR. Copan liquid Stuarts, Copan Amies Gel and Copan Amies Gel with charcoal swabs were all found to give equivalent results for sensitivity and PCR inhibition.

**Claims**

**1.** Method of detecting the presence or absence of methicillin-resistant *S. aureus* (MRSA) in a sample, the method comprising

(a) performing an amplifying step comprising contacting the sample with a set of MRSA primers to produce an amplification product if MRSA is present in the sample,
(b) performing a hybridizing step comprising contacting the amplification product of step (a) with a pair of MRSA probes, wherein a first MRSA probe of the pair of MRSA probes is labeled with a donor fluorescent moiety and wherein a second MRSA probe of the pair of MRSA probes is labeled with a corresponding acceptor fluorescent moiety; and
(c) detecting the presence or absence of fluorescence resonance energy transfer (FRET) between the donor fluorescent moiety of the first MRSA probe and the acceptor fluorescent moiety of the second MRSA probe, wherein the presence of FRET is indicative of the presence of MRSA in the sample and wherein the absence of FRET is indicative of the absence of MRSA in the sample,
wherein the method is capable of detecting each of Staphylococcal Chromosomal Cassettes (SCC*mec*) types I to V of MRSA, and
wherein the set of primers comprises

(i) a primer specific for MRSA type RE2, namely a primer consisting of the nucleic acid sequence 5'- TGA AAT GAA AGA CTG CGG AG -3' (MRSA direct RE2 fwd; SEQ ID NO: 92);
and
(ii) a primer specific for MRSA type RE3, namely a primer consisting of the nucleic acid sequence 5'- CCA CAT CTC ATT AAA TTT TTA AAT TAT ACA C -3' (MRSA direct RE3 fwd; SEQ ID NO: 93);
and

(iii) a primer specific for MRSA type RE7, namely a primer consisting of the nucleic acid sequence 5'- CAA TCC TTT TTA TAT TTA AAA TAT ATT ATA CAC -3' (MRSA direct RE7 fwd; SEQ ID NO: 94).

**2.** The method of claim 1, wherein the set of primers additionally comprises a further primer specific for methicillin-resistant *S. aureus* (MRSA) as well as methicillin-sensitive *S. aureus* (MSSA).

**3.** The method of claim 2, wherein the further primer comprises or consists of the nucleic acid sequence 5'- CAA GGA AAG ATG CTA TCT TCC G -3' (MRSA direct rev; SEQ ID NO: 95).

**4.** The method of any of claims 1 or 3,

- wherein the set of primer consists of at most 4 or 5 primers and/or
- wherein the set of primer comprises or consists of MRSA direct RE2 fwd, MRSA direct RE3 fwd and MRSA direct RE7 fwd and a reverse primer.

**5.** The method of claims any of claims 1 to 4, wherein the reverse primer is MRSA direct rev.

**6.** The method of claims any of claims 1 to 5, wherein at least one probe of the pair of probes comprises or consists of a fluorescent moiety and a nucleic acid sequences selected from the group consisting of

- 5'- AAG TCG CTT TGC CCT TGG G -3' (MRSA direct Fluos w/o label; SEQ ID NO: 96), and
- 5'- CAT GCG TTG GTT CGA TTC TTG -3' (MRSA direct Red 610 w/o label; SEQ ID NO: 97).

**7.** The method of claims any of claims 1 to 6, wherein the first probe including the first label is 5'- AAG TCG CTT TGC CCT TGG G -3'-fluorescein (MRSA direct Fluos) and wherein the second probe including the second labels is LC-Red 610-5'- CAT GCG TTG GTT CGA TTC TTG -3' (MRSA direct Red 610).

**8.** The method of any of claims 1 to 7,

- wherein the members of the pair of MRSA probes hybridize to the amplification product within no more than five, four or three nucleotides;
and/or
- wherein

(a) the donor fluorescent moiety is fluorescein; and/or
(b) wherein the acceptor fluorescent moiety is selected from the group consisting of LC-Red 610, LC-Red 640, LC-Red 670, LC-Red 705, Cy5, and Cy5.5.

**9.** A method of detecting the presence or absence of methicillin-resistant *S. aureus* (MRSA) in a sample, the method comprising

(a) performing an amplifying step comprising contacting the sample with a set of MRSA primers to produce an amplification product if MRSA is present in the sample,
(b) performing a hybridizing step comprising contacting the amplification product of step (a) with a MRSA probe, wherein the MRSA probe is labeled with a donor fluorescent moiety and with a corresponding acceptor fluorescent moiety; and
(c) detecting the presence or absence of fluorescence resonance energy transfer (FRET) between the donor fluorescent moiety and the acceptor fluorescent moiety of the MRSA probe, wherein the presence or absence of FRET is indicative of the presence or absence of MRSA in the sample,
wherein the method is capable of detecting each of Staphylococcal Chromosomal Cassettes (SCC*mec)* types I to V of MRSA, and wherein the set of primers is as defined in claims 1 to 6.

**10.** The method of claim 9,

a) wherein the probe comprises or consists of two fluorescent moieties,
b) wherein the probe comprises or consists of two fluorescent moieties,
wherein the donor fluorescent moiety is fluorescein; and/or wherein the acceptor fluorescent moiety is selected from the group consisting of LC-Red 610, LC-Red 640, LC-Red 670, LC-Red 705, Cy5, and Cy5.5;

c) wherein the amplifying step employs a polymerase enzyme having 5' to 3' exonuclease activity;
d) wherein the donor and acceptor fluorescent moieties are within no more than 5 nucleotides of each other on the probe;
e) wherein the probe comprises a nucleic acid sequence that permits secondary structure formation, wherein the secondary structure formation results in spatial proximity between the donor and acceptor fluorescent moiety; and/or
f) wherein the acceptor fluorescent moiety is a quencher.

**11.** The method of any of claims 1 to 10,

a) wherein the detecting step comprises exciting the sample at a wavelength absorbed by the donor fluorescent moiety and visualizing and/or measuring the wavelength emitted by the acceptor fluorescent moiety;
b) wherein the detecting comprises quantitating the FRET;
c) wherein the presence of the FRET within 55, 45 or 35 cycles is indicative of the presence of MRSA in the sample;
d) wherein the detecting step is performed after each step amplification and hybridization and/or in real-time;
e) further comprising determining the melting temperature between one or both of the probe(s) and the amplification product of step (a), wherein the melting temperature confirms the presence or the absence of MRSA;
f) further comprising:

preventing amplification of a contaminant nucleic acid;

g) further comprising:

preventing amplification of a contaminant nucleic acid by performing the amplification step (a) in the presence of uracil and treating the sample with uracil-DNA glycosylase prior to a first amplifying step;

h) wherein the method is performed on a control sample;
i) wherein the sample is a biological sample; and/or
j) wherein the sample is selected from the group consisting of a swab of an infected wound, a skin swab, a nasal swab, a throat swab, a groin swab, a axilla swab, a swab from site of an invasive device, a swab from site of possible infection, a body fluid, a blood sample, a urine sample and a perineum swab.

**12.** A composition comprising a set of primers as defined in any of claims 1 to 5.

**13.** The composition of claim 12 further comprising a probe and/or a pair of probes as defined in any of claims 6 to 8 or 12.

**14.** A kit comprising:

(a) a set of primers as defined in any of claims 1 to 5;
(b) a pair of probes as defined in claim 6 or 8 or a probe as defined in claim 10;
and
(c) a donor fluorescent moiety and a corresponding fluorescent moiety labeling the probe(s),
wherein the kit is capable of detecting each of Staphylococcal Chromosomal Cassettes (SCC*mec*) types I to V of MRSA,

**15.** The kit of claim 14,

a) being suitable to carry out the method of any of claims 1 to 11;
b) further comprising a package label or package insert having instructions thereon for using the set of MRSA primers and the pair of MRSA probes to detect the presence or absence of in a sample; and/or
c) further comprising uracil-DNA-glycosylase and/or a DNA polymerase and/or a suitable buffer.

## Patentansprüche

**1.** Verfahren zum Nachweisen der Gegenwart oder Abwesenheit von Methicillinresistentem *S. aureus* (MRSA) in einer Probe, wobei das Verfahren umfasst

(a) Durchführen eines Amplifizierungsschrittes umfassend das Inkontaktbringen der Probe mit einem Satz von MRSA-Primern, um ein Amplifikationsprodukt herzustellen, wenn MRSA in der Probe vorhanden ist,
(b) Durchführen eines Hybridisierungsschritts umfassend das Inkontaktbringen des Amplifikationsprodukts aus Schritt (a) mit einem MRSA-Sondenpaar, wobei die erste MRSA-Sonde des MRSA-Sondenpaars mit einer Donorfluoreszenzkomponente markiert ist und wobei eine zweite MRSA-Sonde des MRSA-Sondenpaars mit einer entsprechenden Akzeptorfluoreszenzkomponente markiert ist; und
(c) Nachweisen der Gegenwart oder Abwesenheit von Fluoreszenzresonanz-Energietransfer (FRET) zwischen der Donorfluoreszenzkomponente der ersten MRSA-Sonde und der Akzeptorfluoresenzkomponente der zweiten MRSA-Sonde, wobei die Gegenwart von FRET für die Gegenwart von MRSA in der Probe indikativ ist und wobei die Abwesenheit von FRET für die Abwesenheit von MRSA in der Probe indikativ ist,
wobei das Verfahren zum Nachweisen jeder der Staphylococcus-Chromosomalkassetten (Staphylococcal Chromosomal Cassettes; SCC*mec*) der Typen I bis V von MRSA in der Lage ist, und
wobei der Primersatz umfasst

(i) einen für MRSA Typ RE2 spezifischen Primer, nämlich einen Primer bestehend aus der Nukleinsäuresequenz 5'- TGA AAT GAA AGA CTG CGG AG -3' (MRSA direct RE2 fwd; SEQ ID NO: 92);
und
(ii) einen für MRSA Typ RE3 spezifischen Primer, nämlich einen Primer bestehend aus der Nukleinsäuresequenz 5'- CCA CAT CTC ATT AAA TTT TTA AAT TAT ACA C -3' (MRSA direct RE3 fwd; SEQ ID NO: 93);
und
(iii) einen für MRSA Typ RE7 spezifischen Primer, nämlich einen Primer bestehend aus der Nukleinsäuresequenz 5'- CAA TCC TTT TTA TAT TTA AAA TAT ATT ATA CAC -3‘ (MRSA direct RE7 fwd; SEQ ID NO: 94).

**2.** Verfahren nach Anspruch 1, wobei der Primersatz zusätzlich einen weiteren Primer enthält, der sowohl für Methicillin-resistenten *S. aureus* (MRSA) wie auch Methicillin-sensitiven *S. aureus* (MSSA) spezifisch ist.

**3.** Verfahren nach Anspruch 2, wobei der zusätzliche Primer die Nukleinsäuresequenz 5'- CAA GGA AAG ATG CTA TCT TCC G -3' (MRSA direct rev; SEQ ID NO: 95) umfasst oder daraus besteht.

**4.** Verfahren nach einem beliebigen der Ansprüche 1 bis 3,

- wobei der Primersatz aus höchstens 4 oder 5 Primern besteht und/oder
- wobei der Primersatz MRSA direct RE2 fwd, MRSA direct RE3 fwd und MRSA direct RE7 fwd und einen reversen Primer umfasst oder daraus besteht.

**5.** Verfahren nach einem beliebigen der Ansprüche 1 bis 4, wobei der reverse Primer MRSA direct rev ist.

**6.** Verfahren nach einem beliebigen der Ansprüche 1 bis 5, wobei mindestens eine Sonde des Sondenpaars eine Fluoreszenzkomponente und eine Nukleinsäuresequenz ausgewählt aus der Gruppe bestehend aus

- 5'- AAG TCG CTT TGC CCT TGG G -3' (MRSA direct Fluos w/o label; SEQ ID NO: 96), und
- 5'- CAT GCG TTG GTT CGA TTC TTG -3' (MRSA direct Red 610 w/o label; SEQ ID NO: 97)
enthält oder daraus besteht.

**7.** Verfahren nach einem beliebigen der Ansprüche 1 bis 6, wobei die erste Probe einschließlich des ersten Markers 5'- AAG TCG CTT TGC CCT TGG G -3'-Fluorescein (MRSA direct Fluos) ist, und wobei die zweite Sonde einschließlich des zweiten Labels LC-Red 610 -5' - CAT GCG TTG GTT CGA TTC TTG -3' (MRSA direct Red 610) ist.

**8.** Verfahren nach einem beliebigen der Ansprüche 1 bis 7,

- wobei die Mitglieder des MRSA-Sondenpaars innerhalb von höchstens fiinf, vier oder drei Nukleotiden an das Amplifikationsprodukt hybridisieren;
und/oder
- wobei

(a) die Donorfluoreszenzkomponente Fluoreszein ist; und/oder
(b) die Akzeptorfluoreszenzkomponente ausgewählt ist aus der Gruppe bestehend aus LC-Red 610, LC-

Red 640, LC-Red 670, LC-Red 705, Cy5 und Cy5.5 ist.

**9.** Verfahren zum Nachweisen der Gegenwart oder Abwesenheit von Methicillinresistentem *S. aureus* (MRSA) in einer Probe, wobei das Verfahren umfasst

(a) Durchführen eines Amplifizierungsschrittes umfassend das Inkontaktbringen der Probe mit einem Satz von MRSA-Primern, um ein Amplifikationsprodukt herzustellen, wenn MRSA in der Probe vorhanden ist,
(b) Durchführen eines Hybridisierungsschritts umfassend das Inkontaktbringen des Amplifikationsprodukts aus Schritt (a) mit einer MRSA-Sonde, wobei die MRSA-Sonde mit einer Donorfluoreszenzkomponente und mit einer entsprechenden Akzeptorfluoreszenzkomponente markiert ist; und
(c) Nachweisen der Gegenwart oder Abwesenheit von Fluoreszenzresonanz-Energietransfer (FRET) zwischen der Donorfluoreszenzkomponente und der Akzeptorfluoreszenzkomponente der MRSA-Sonde, wobei die Gegenwart oder Abwesenheit von FRET indikativ für die Gegenwart bzw. Abwesenheit von MRSA in der Sonde ist, wobei das Verfahren zum Nachweisen jeder der Staphylococcus-Chromosomalkassetten (Staphylococcal Chromosomal Cassettes; SCCmec) der Typen I bis V von MRSA in der Lage ist, und wobei der Primersatz wie in den Ansprüchen 1 bis 6 definiert ist.

**10.** Verfahren nach Anspruch 9,

a) wobei die Sonde zwei Fluoreszenzkomponenten umfasst oder daraus besteht,
b) wobei die Sonde zwei Fluoreszenzkomponenten umfasst oder daraus besteht, wobei die Donorfluoreszenzkomponente Fluoreszein ist; und/oder wobei die Akzeptorfluoreszenzkomponente ausgewählt ist aus der Gruppe bestehend aus LC-Red 610, LC-Red 640, LC-Red 670, LC-Red 705, Cy5 und Cy5.5;
c) wobei der Amplifizierungsschritt ein Polymeraseenzym mit 5' nach 3' Exonukleaseaktivität einsetzt;
d) wobei die Donor- und Akzeptorfluoreszenzkomponenten innerhalb von höchstens 5 Nukleotiden voneinander auf der Sonde sind;
e) wobei die Sonde eine Nukleinsäuresequenz umfasst, die die Ausbildung einer Sekundärformation erlaubt, wobei die Ausbildung der Sekundärformation in der räumlichen Nähe der Donor- und Akzeptorfluoreszenzkomponente resultiert und/oder
f) wobei die Akzeptorfluoreszenzkomponente ein Quencher ist.

**11.** Verfahren nach einem beliebigen der Ansprüche 1 bis 10,

a) wobei der Nachweisschritt das Anregen der Probe mit einer Wellenlänge, die von der Donorfluoreszenzkomponente absorbiert wird, und das Sichtbarmachen und/oder Messen der von der Akzeptorfluoreszenzkomponente emittierten Wellenläge umfasst;
b) wobei das Nachweisen das Quantifizieren von FRET umfasst;
c) wobei die Gegenwart von FRET innerhalb von 55, 45 oder 35 Zyklen indikativ für die Gegenwart von MRSA in der Probe ist;
d) wobei der Nachweisschritt nach jedem Amplifizierungs- und Hybridisierungsschritt und/oder in Echtzeit durchgeführt wird;
e) weiterhin umfassend das Bestimmen der Schmelztemperatur zwischen einer oder beiden Sonde(n) und dem Amplifikationsprodukt von Schritt (a), wobei die Schmelztemperatur die Gegenwart oder Abwesenheit von MRSA bestätigt;
f) weiterhin umfassend: Verhindern der Amplifikation einer kontaminierenden Nukleinsäure;
g) weiterhin umfassend: Verhindern der Amplifikation einer kontaminierenden Nukleinsäure durch Durchführen des Amplifizierungsschrittes (a) in Gegenwart von Uracil und Behandeln der Probe mit Uracil-DNA-Glycosylase vor dem ersten Amplifizierungsschritt;
h) wobei das Verfahren mit einer Kontrollprobe durchgeführt wird;
i) wobei die Probe eine biologische Probe ist; und/oder
j) wobei die Probe ausgewählt ist aus der Gruppe bestehend aus einem Abstrich einer infizierten Wunde, einem Hautabstrich, einem Nasenabstrich, einem Halsabstrich, einem Leistenabstrich, einem Achselabstrich, einem Abstrich einer Stelle eines invasiven Geräts, einem Abstrich einer Stelle einer möglichen Infektion, einer Körperflüssigkeit, einer Blutprobe, einer Urinprobe oder einem Darmabstrich.

**12.** Zusammensetzung, umfassend einen wie in einem beliebigen der Ansprüche 1 bis 5 definierten Primersatz.

**13.** Zusammensetzung nach Anspruch 12, weiterhin umfassend eine Probe und/oder ein Probenpaar wie in einem beliebigen der Ansprüche 6 bis 8 oder 12 definiert.

**14.** Kit, umfassend

(a) einen Primersatz wie in einem beliebigen der Ansprüche 1 bis 5 definiert;
(b) einen Sondensatz wie in Anspruch 6 oder 8 definiert oder eine Probe wie in Anspruch 10 definiert; und
(c) eine Donorfluoreszenzkomponente und eine entsprechende, die Sonde(n) markierende Fluoreszenzkomponente,
wobei der Kit zum Nachweisen jeder der Staphylococcus-Chromosomalkassetten (Staphylococcal Chromosomal Cassettes; SCC*mec*) der Typen I bis V von MRSA in der Lage ist.

**15.** Kit nach Anspruch 14,

(a) geeignet, das Verfahren nach einem beliebigen der Ansprüche 1 bis 11 durchzuführen;
(b) weiterhin umfassend eine Packungsmarkierung oder einen Beipackzettel mit darauf befindlichen Anweisungen zur Verwendung des MRSA-Primersatzes und des MRSA-Sondenpaars zum Nachweisen der Gegenwart oder Abwesenheit in einer Probe und/oder
(c) weiterhin umfassend Uracil-DNA-Glycolase und/oder eine DNA-Polymerase und/oder einen geeigneten Puffer.

## Revendications

**1.** Procédé de détection de la présence ou de l'absence d'un *S. aureus* résistant à la méthicilline (MRSA) dans un échantillon, le procédé comprenant

(a) la réalisation d'une étape d'amplification comprenant la mise en contact de l'échantillon avec un jeu d'amorces pour MRSA afin de produire un produit d'amplification si un MRSA est présent dans l'échantillon,
(b) la réalisation d'une étape d'hybridation comprenant la mise en contact du produit d'amplification de l'étape (a) avec une paire de sondes de MRSA, dans laquelle une première sonde de MRSA de la paire de sondes de MRSA est marquée avec une fraction fluorescente donneur et dans laquelle une deuxième sonde de MRSA de la paire de sondes de MRSA est marquée avec une fraction fluorescente accepteur correspondante ; et
(c) la détection de la présence ou de l'absence d'un transfert d'énergie par résonance de fluorescence (FRET) entre la fraction fluorescente donneur de la première sonde de MRSA et la fraction fluorescente accepteur de la deuxième sonde de MRSA, dans laquelle la présence d'un FRET est indicatrice de la présence d'un MRSA dans l'échantillon, et dans laquelle l'absence de FRET est indicatrice de l'absence de MRSA dans l'échantillon,
dans lequel le procédé est capable de détecter chacun des types 1 à V des cassettes chromosomiques staphylococciques (SCCmec) de MRSA, et
dans lequel le jeu d'amorces comprend

(i) une amorce spécifique du type RE2 de MRSA, à savoir une amorce constituée de la séquence d'acide nucléique 5'- TGA AAT GAA AGA CTG CGG AG -3' (MRSA direct RE2 sens ; SEQ ID NO: 92) ;
et
(ii) une amorce spécifique du type RE3 de MRSA, à savoir une amorce constituée de la séquence d'acide nucléique 5'- CCA CAT CTC ATT AAA TTT TTA AAT TAT ACA C -3' (MRSA direct RE3 sens ; SEQ ID NO: 93) ;
et
(iii) une amorce spécifique du type RE7 de MRSA, à savoir une amorce constituée de la séquence d'acide nucléique 5'- CAA TCC TTT TTA TAT TTA AAA TAT ATT ATA CAC -3' (MRSA direct RE7 sens; SEQ ID NO: 94).

**2.** Procédé selon la revendication 1, dans lequel le jeu d'amorces comprend en outre une amorce supplémentaire spécifique de *S. aureus* résistant à la méthicilline (MRSA) ainsi que de *S. aureus* sensible à la méthicilline (MSSA).

**3.** Procédé selon la revendication 2, dans lequel l'amorce supplémentaire comprend ou est constituée de la séquence d'acide nucléique 5'- CAA GGA AAG ATG CTA TCT TCC G -3' (MRSA direct antisens ; SEQ ID NO: 95).

**4.** Procédé selon l'une quelconque des revendications 1 ou 3,

- dans lequel le jeu d'amorce est constitué d'au plus 4 ou 5 amorces et/ou
- dans lequel le jeu d'amorce comprend ou est constitué de MRSA direct RE2 sens, MRSA direct RE3 sens et MRSA direct RE7 sens et d'une amorce anti-sens.

**5.** Procédé selon l'une quelconque des revendications 1 à 4, dans lequel l'amorce anti-sens est MRSA direct anti-sens.

**6.** Procédé selon l'une quelconque des revendications 1 à 5, dans lequel au moins une sonde de la paire de sondes comprend ou est constituée d'une fraction fluorescente et d'une séquence d'acide nucléique choisie dans le groupe constitué par

- 5'- AAG TCG CTT TGC CCT TGG G -3' (MRSA direct Fluos sans étiquette ; SEQ ID NO: 96), et
- 5'- CAT GCG TTG GTT CGA TTC TTG -3' (MRSA direct Red 610 sans étiquette ; SEQ ID NO: 97).

**7.** Procédé selon l'une quelconque des revendications 1 à 6, dans lequel la première sonde comprenant la première étiquette est 5'- AAG TCG CTT TGC CCT TGG G -3'-fluorescéine (MRSA direct Fluos), et dans lequel la deuxième sonde comprenant la deuxième étiquette est LC-Red 610-5'- CAT GCG TTG GTT CGA TTC TTG -3' (MRSA direct Red 610).

**8.** Procédé selon l'une quelconque des revendications 1 à 7,

- dans lequel les éléments de la paire de sondes de MRSA s'hybrident au produit d'amplification dans la limite de pas plus de cinq, quatre ou trois nucléotides ;
et/ou
- dans lequel

(a) la fraction fluorescente donneur est la fluorescéine ; et/ou
(b) dans lequel la fraction fluorescente accepteur est choisie dans le groupe constitué par le LC-Red 610, LC-Red 640, LC-Red 670, LC-Red 705, Cy5, et Cy5.5.

**9.** Procédé de détection de la présence ou de l'absence d'un *S. aureus* résistant à la méthicilline (MRSA) dans un échantillon, le procédé comprenant

(a) la réalisation d'une étape d'amplification comprenant la mise en contact de l'échantillon avec un jeu d'amorces pour MRSA afin de produire un produit d'amplification si un MRSA est présent dans l'échantillon,
(b) la réalisation d'une étape d'hybridation comprenant la mise en contact du produit d'amplification de l'étape (a) avec une sonde de MRSA, dans laquelle la sonde de MRSA est marquée avec une fraction fluorescente donneur et avec une fraction fluorescente accepteur correspondante ; et
(c) la détection de la présence ou de l'absence d'un transfert d'énergie par résonance de fluorescence (FRET) entre la fraction fluorescente donneur et la fraction fluorescente accepteur de la sonde de MRSA, dans laquelle la présence ou l'absence de FRET est indicatrice de la présence ou de l'absence de MRSA dans l'échantillon, dans lequel le procédé est capable de détecter chacun des types I à V des cassettes chromosomiques staphylococciques (SCCmec) de MRSA, et dans lequel le jeu d'amorces est tel que défini selon les revendications 1 à 6.

**10.** Procédé selon la revendication 9,

a) dans lequel la sonde comprend ou est constituée de deux fractions fluorescentes,
b) dans lequel la sonde comprend ou est constituée de deux fractions fluorescentes,
dans laquelle la fraction fluorescente donneur est la fluorescéine ; et/ou dans laquelle la fraction fluorescente accepteur est choisie dans le groupe constitué par le LC-Red 610, LC-Red 640, LC-Red 670, LC-Red 705, Cy5, et Cy5.5 ;
c) dans lequel l'étape d'amplification emploie une enzyme polymérase ayant une activité exonucléase 5' vers 3' ;
d) dans lequel les fractions fluorescentes donneur et accepteur sont à pas plus de 5 nucléotides l'une de l'autre sur la sonde ;
e) dans lequel la sonde comprend une séquence d'acide nucléique qui permet la formation d'une structure secondaire, dans lequel la formation d'une structure secondaire entraîne une proximité spatiale entre la fraction fluorescente donneur et accepteur ; et/ou
f) dans lequel la fraction fluorescente accepteur est un extincteur.

**11.** Procédé selon l'une quelconque des revendications 1 à 10,

a) dans lequel l'étape de détection comprend l'excitation de l'échantillon à une longueur d'onde absorbée par la fraction fluorescente donneur et la visualisation et/ou la mesure de la longueur d'onde émise par la fraction fluorescente accepteur ;
b) dans lequel la détection comprend la quantification du FRET ;
c) dans lequel la présence du FRET dans la limite de 55, 45 ou 35 cycles est indicatrice de la présence de MRSA dans l'échantillon ;
d) dans lequel l'étape de détection est réalisée après chaque étape d'amplification et d'hybridation et/ou en temps réel ;
e) comprenant en outre la détermination de la température de fusion entre une ou les deux sonde(s) et le produit d'amplification de l'étape (a), dans lequel la température de fusion confirme la présence ou l'absence de MRSA;
f) comprenant en outre :

la prévention de l'amplification d'un acide nucléique contaminant ;

g) comprenant en outre :

la prévention de l'amplification d'un acide nucléique contaminant en effectuant l'étape d'amplification (a) en présence d'uracile et le traitement de l'échantillon avec de l'uracile-ADN glycosylase avant une première étape d'amplification ;

h) dans lequel le procédé est effectué sur un échantillon témoin ;
i) dans lequel l'échantillon est un échantillon biologique ; et/ou
j) dans lequel l'échantillon est choisi dans le groupe constitué par un écouvillonnage d'une plaie infectée, un écouvillonnage cutané, un écouvillonnage nasal, un écouvillonnage de gorge, un écouvillonnage de l'aine, un écouvillonnage axillaire, un écouvillonnage sur le site d'un dispositif invasif, un écouvillonnage sur le site d'une infection possible, un fluide corporel, un échantillon de sang, un échantillon d'urine et un écouvillon du périnée.

**12.** Composition comprenant un jeu d'amorces tel que défini selon l'une quelconque des revendications 1 à 5.

**13.** Composition selon la revendication 12, comprenant en outre une sonde et/ou une paire de sondes selon l'une quelconque des revendications 6 à 8 ou 12.

**14.** Kit comprenant :

(a) un jeu d'amorces selon l'une quelconque des revendications 1 à 5 ;
(b) une paire de sondes selon la revendication 6 ou 8 ou une sonde selon la revendication 10 ; et
(c) une fraction fluorescente donneur et une fraction fluorescente correspondant marquant la(les) sonde(s), dans laquelle le kit est en mesure de détecter chacun des types I à V des cassettes chromosomiques staphylococciques (SCCmec) de MRSA.

**15.** Kit selon la revendication 14,

a) étant approprié pour exécuter le procédé selon l'une quelconque des revendications 1 à 11 ;
b) comprenant en outre une étiquette d'emballage ou une notice d'emballage comportant sur lui des instructions pour l'utilisation du jeu d'amorces de MRSA et de la paire de sondes de MRSA afin de détecter la présence ou l'absence dans un échantillon; et/ou
c) comprenant en outre une uracile-ADN-glycosylase et/ou une ADN polymérase et/ou un tampon approprié.

**RE2:**

TTTGCTTCACTATAAGTATTCAGTATAAAGAATATTTCGCTATTATTTACTT
GAAATGAAAGACTGCGGAGGCTAACTATGTCAAAAATCATGAACCTCATTAC
TTATGATAAGCTTCTTAAAAACATAACAGCAATTCACATAAACCTCATATGT
TCTGATACATTCAAAATCCCTTTATGAAGCGGCTGAAAAAACCGCATCATTT
GATATGCTTCTTAAAAACATAACAGCAATTCACATAAACCTCATATGTTCTG
ATACATTCAAAATCCCTTTATGAAGCGGCTGAAAAAACCGCATCATTTATGA
TATGCTTCTCC **orfX →**

**RE3:**

CATTCTTTCTTGATTCCATTAGTTTAAATTTAAAATTTCATCATCAATTTCT
TAATTTAATTGTAGTTCCATAATCAATATAATTTGTACAGTTATTATATATT
CTAGATCATCAATAGTTGAAAAATGGTTTATTAAACACTCTATAAACATCGT
ATGATATTGCAAGGTATAATCCAATATTTCATATATGTAATTCCTCCACATC
TCATTAAATTTTTAAATTATACACAACCTAATTTTTAGTTTTATTTATGATA
CGCTTCTCC **orfX →**

**RE7:**

CAAAAAATATATTTACTTTAGTCAAATCATCTTCACTAGTGTAATTATCGAA
TGATTTATAACTAACATTTTCTAATTTATTTAACATAAAATCAATCCTTTTT
ATATTTAAAATATATTATACACAATCCGTTTTTAGTTTTATTTATGATACG
CCTCTCC **orfX →**

**orfX (SEQ ID NO:77):**

CCACGCATAATCTTAAATGCTCTGTACACTTGTTCAATTAACACAACCCGCA
TCATTTGATGTGGGAATGTCATTTTGCTGAATGATAGTGCGTAGTTACTGCG
TTGTAAGACGTCCTTGTGCAGGCCGTTTGATCCGCCAATGACGAATACAAAG
TCGCTTTGCCCTTGGGTCATGCGTTGGTTCAATTCTTGGGCCAATCCTTCGG
AAGATAGCATCTTTCCTTGTATTTCTAATGTAATGACTGTTGATTGT

**Fig. 1**

(SEQ ID NO: 86)

```
TTTGCTTCAC TATAAGTATT CAGTATAAAG AATATTTCGC TATTATTTAC
                      G(1)
TTGAAATGAA AGACTGCGGA GGCTAACTAT GTCAAAAATC ATGAACCTCA

TTACTTATGA TAAGCTTCTT AAAAACATAA CAGCAATTCA CATAAACCTC

ATATGTTCTG ATACATTCAA AATCCCTTTA TGAAGCGGCT GAAAAAACCG

CATCATTTGA TATGCTTCTT AAAAACATAA CAGCAATTCA CATAAACCTC

ATATGTTCTG ATACATTCAA AATCCCTTTA TGAAGCGGCT GAAAAAACCG

CATCATTTAT GATATGCTTC TCCACGCATA ATCTTAAATG CTCTATACAC
                       (3)TG(1)         C(1)   G(7)
TTGCTCAATT AACACAACCC GCATCATTTG ATGTGGGAAT GTCATTTTGC
   T(8)                                A(2)
TGAATGATAG TGCGTAGTTA CTGCGTTGTA AGACGTCCTT GTGCAGGCCG
  A(1)          A(1)       A(3)
TTTGATCCGC CAATGACGAA TACAAAGTCG CTTTGCCCTT GGGTCATGCG
                      A(3)
TTGGTTCAAT TCTTGGGCCA ATCCTTCGGA AGATAGCATC TTTCCTTGTA
                 T(1)
TTTCTAATGT AATGACTGTG GATTGTGGTT TAATTTTGGC TAGTATTCGT
                     T(3)        (9)G :(1)
TGGCCTTCTT TTTCTTTTAC TTGCTCAATT TCTTTGTCGC TCA
                            G(2)
```

**Fig. 2**

Fig. 3

A

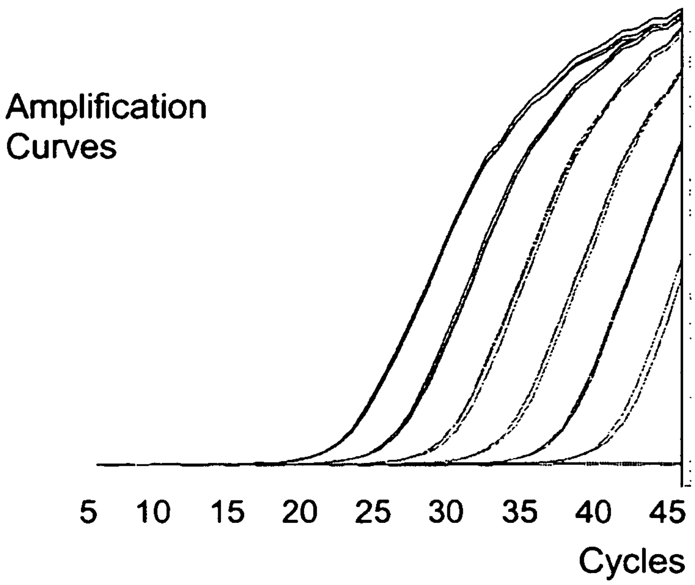
Amplification
Curves
5 10 15 20 25 30 35 40 45
Cycles

B

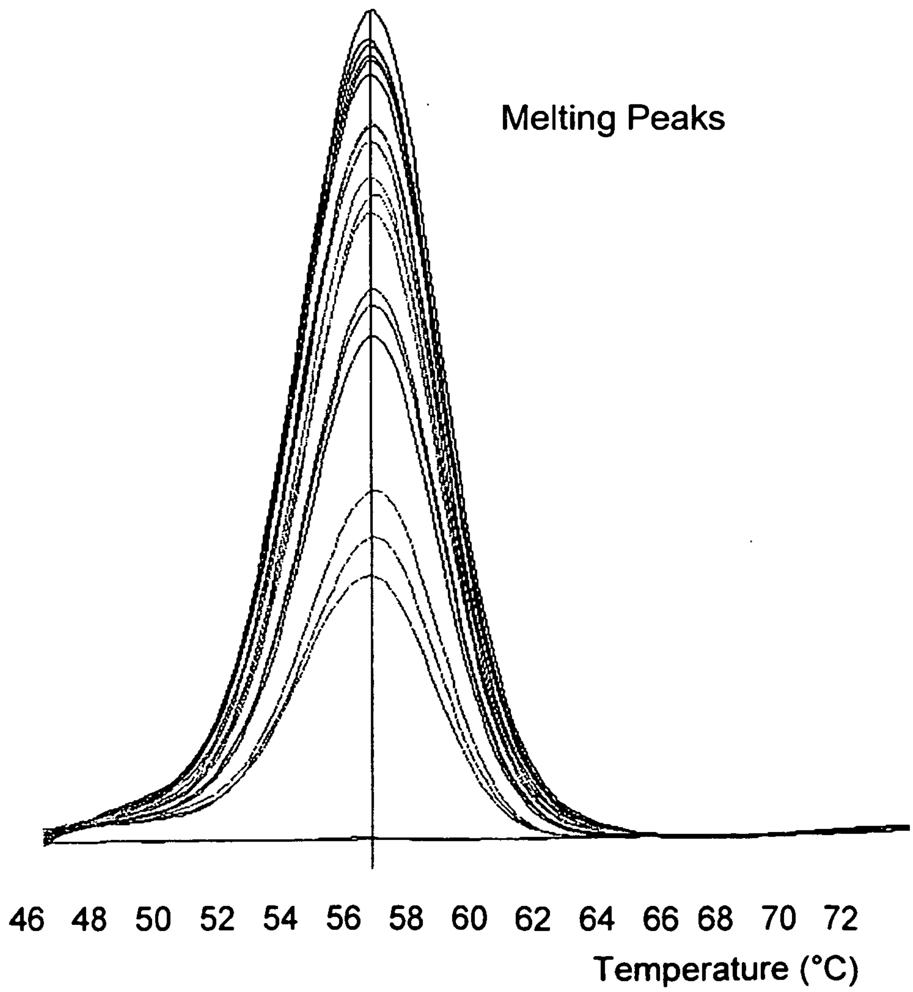
Melting Peaks
46 48 50 52 54 56 58 60 62 64 66 68 70 72
Temperature (°C)

Fig. 4

A

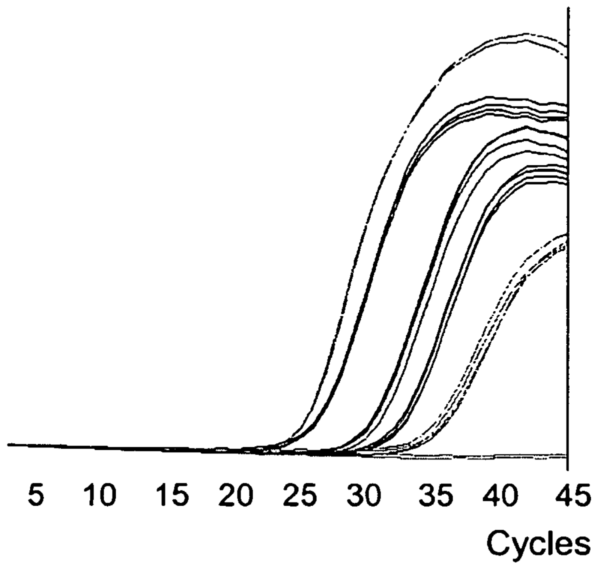
5
10
15
20
25
30
35
40
45
Cycles

B

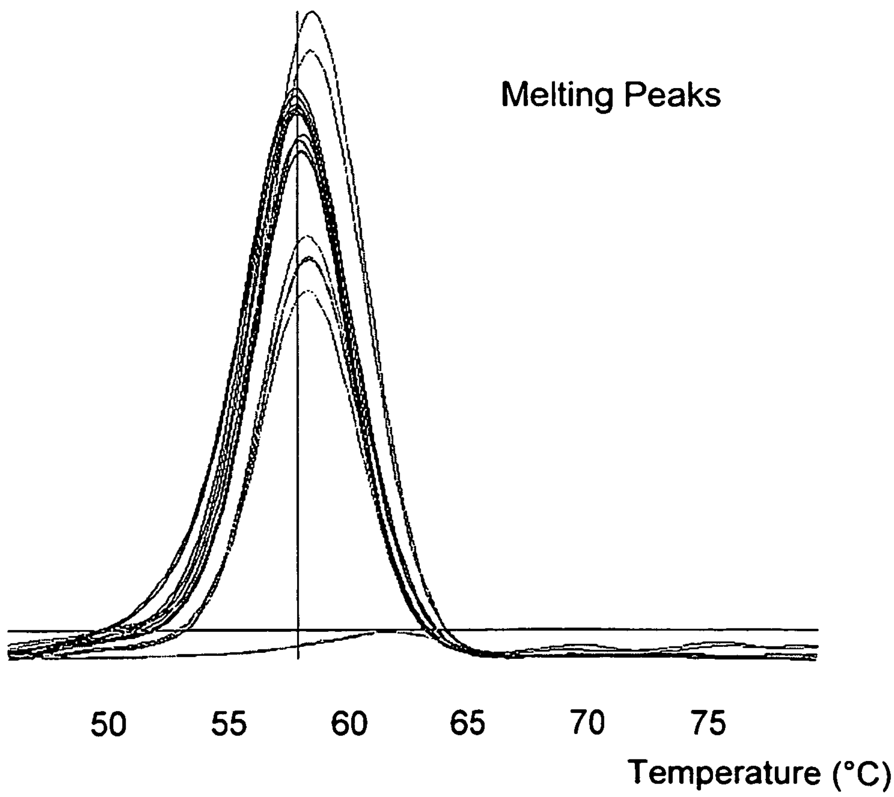
Melting Peaks
50
55
60
65
70
75
Temperature (°C)

Fig. 5

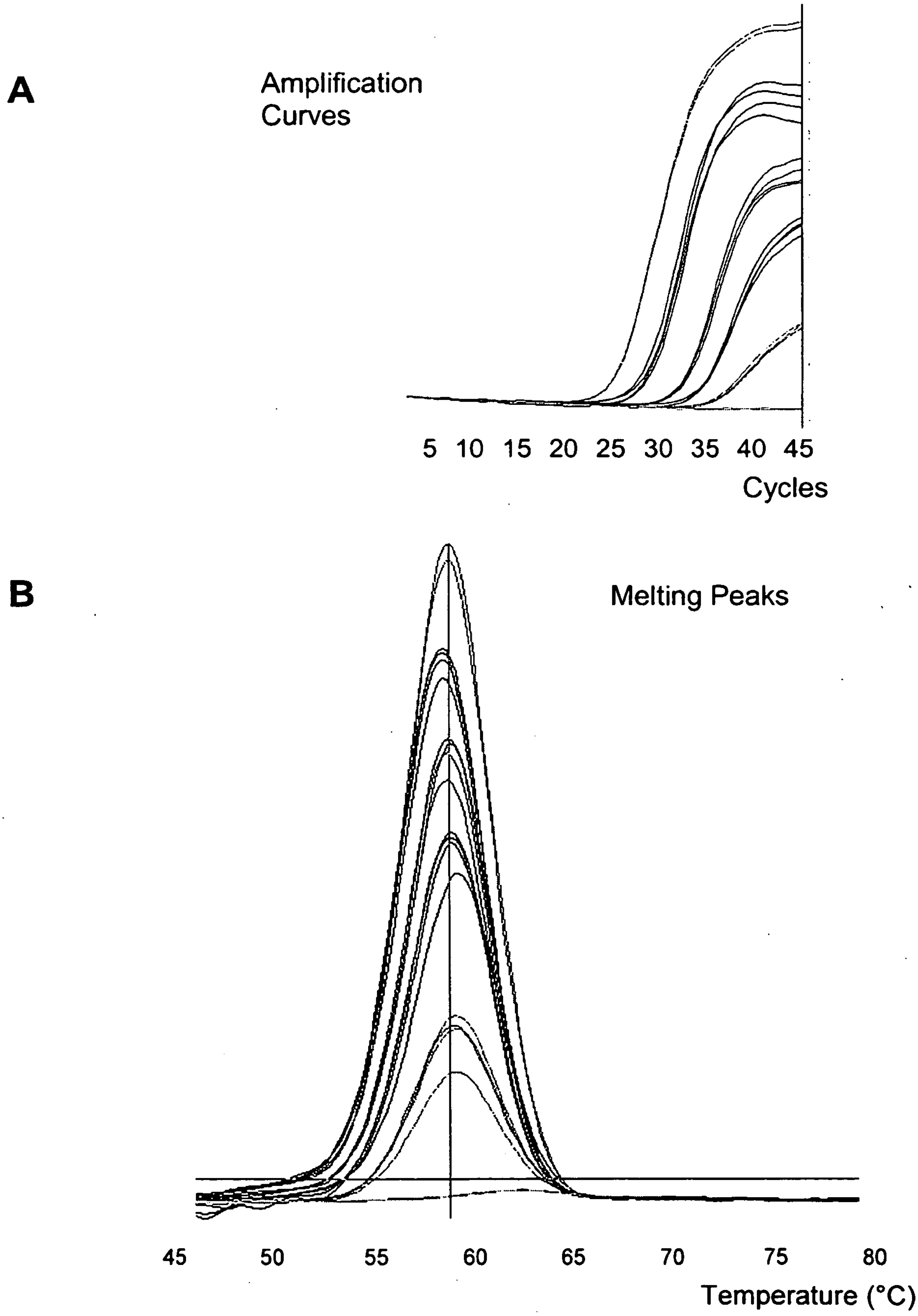
A
Amplification
Curves
5 10 15 20 25 30 35 40 45
Cycles
B
Melting Peaks
45 50 55 60 65 70 75 80
Temperature (°C)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1529847 A
- WO 0212263 A
- US 4996143 A
- US 5565322 A
- US 5849489 A
- US 6162603 A
- WO 8403285 A
- US 6174670 B
- US 4683202 A
- US 4683195 A
- US 4800159 A
- US 4965188 A
- WO 9746707 A
- WO 9746714 A
- WO 9746712 A
- US 5035996 A
- US 5683896 A
- US 5945313 A
- WO 9804730 A
- US 5386024 A
- DE 3734442 A
- WO 0137291 A
- WO 9641811 A
- WO 9916781 A


### Non-patent literature cited in the description


- **JOHNSON AP et al.** *J Antimicrob Chemother,* 2005, vol. 56 (3), 455-62
- **ENRIGHT et al.** *Proc. Nat. Acad. Sci. U.S.A.,* 2002, vol. 99, 7687-7692
- **GUIGNARD B et al.** *Curr Opin Pharmacol,* 2005, vol. 5 (5), 479-89
- **ITO et al.** *Antimicrob Agents Chemother.,* 2004, vol. 48, 2637-2651
- **SMITH ; WATERMAN.** *Adv. Appl. Math.,* 1981, vol. 2, 482
- **PEARSON ; LIPMAN.** *Proc. Natl. Acad. Sci. U.S.A.,* 1988, vol. 85, 2444-2448
- **ALTSCHUL et al.** *J. Mol. Biol.,* 1990, vol. 215, 403-410
- **UHLMANN ; PEYMAN.** *Chemical Reviews,* 1990, vol. 90, 543
- **VERMA S. ; ECKSTEIN F.** *Annu. Rev. Biochem.,* 1998, vol. 67, 99-134
- Diagnostic Molecular Microbiology: Principles and Applications. American Society for Microbiology, 1993
- **UHL et al.** *J Clin Microbiol.,* 2005, vol. 8, 4046-51
- **WALSH.** Enzymatic Reaction Mechanisms. W. H. Freeman and Company, 1979
- **SAMBROOK J. et al.** Molecular Cloning: A Laboratory Manual. Cold Spring Harbor Laboratory Press, 1989
- **VOGELSTEIN et al.** *Proc. Natl. Acad. U.S.A.,* 1979, vol. 76, 615-619
- **MARKO et al.** *Anal. Biochem.,* 1982, vol. 121, 382-387
- **JAKOBI R. et al.** *Anal. Biochem.,* 1988, vol. 175, 196-201
- **ALDERTON et al.** *Anal. Biochem.,* 1992, vol. 201, 166-169